# EUROPÄISCHE PATENTANMELDUNG

(11) **EP 2 112 510 A1**
(43) Veröffentlichungstag der Anmeldung: **28.10.2009**
(21) Anmeldenummer: 09005601.1
(22) Anmeldetag: 21.04.2009
(51) Int. Cl.: G01N 33/50, G01N 33/68

(54) **Verwendung von biologischen Photorezeptoren als direkt lichtgesteuerte lonenkanäle**

(30) Priorität: 22.04.2008 DE 102008020152
(71) Anmelder: Max-Planck-Gesellschaft zur Förderung der Wissenschaften e.V., 80539 München (DE)
(72) Erfinder: Bamberg, Ernst, 60438 Frankfurt/Main (DE); Nagel, Georg, 97082 Würzburg (DE); Hegemann, Peter, 10115 Berlin (DE)
(74) Vertreter: Müller - Hoffmann & Partner

(57) **Zusammenfassung**

Die Erfindung betriff die Verwendung eines biologischen Photorezeptors als direkt lichtgesteuerter Inenkanal zur Veränderung der Ionenleitfähigkeit einer Membran mit Hilfe von Licht, bei welcher der Photorezeptor ein Apoprotein und ein lichtsensitives Polyen aufweist, wobei das lichtsensitive Polyen kovalent an das Apoprotein gebunden ist, mit dem Apoprotein wechselwirkt und als lichtempfindliches Tor fungiert, bei welcher das Apoprotein ein Ionenkanal bildendes Opsin oder ein Derivat oder Fragment eines Ionenkanal bildenden Opsins ist und bei welcher durch Belichtung mittels Veränderung der lonenleitfähigkeit der Membran der Tod einer Zelle hervorgerufen werden kann oder hervorgerufen wird.

## Beschreibung

Die vorliegende Erfindung betrifft die Verwendung von biologischen Photorezeptoren als direkt lichtgesteuerte lonenkanäle.

Es ist seit längerem bekannt, dass eine Vielzahl zentraler Vorgänge in pflanzlichen und tierischen Zellen ganz oder teilweise über Änderungen der intrazellulären Konzentration bestimmter Ionen, beispielsweise der Protonenkonzentration, und Änderungen des Membranpotentials und der lonengradienten über die Membran gesteuert wird. Dementsprechend ist die Aufklärung von Mechanismen der intrazellulären Ionen-Regulation, insbesondere der pH-Regulation, und der Mechanismen von spannungsabhängigen lonenkanälen und lonentransportern Gegenstand ausgedehnter Forschungsaktivitäten. Für solche Forschungen ist eine schnelle und leichte Messung und/oder Manipulation von intrazellulären lonenkonzentrationen, insbesondere Protonenkonzentrationen, sowie von elektrischen Gradienten und lonengradienten, insbesondere Protonengradienten, über Zellmembranen von großem Interesse.

Ein grundsätzlich sehr vorteilhafter Weg zur Ermöglichung solcher Messungen und/oder Manipulationen wäre die Einführung eines lichtgesteuerten lonenkanals in eine solche Membran, um gezielt das Membranpotential und/oder den Fluss bestimmter Ionen, z.B. Protonen, durch die Membran zu verändern.

Im Stand der Technik sind eine Reihe lichtgesteuerter lonentransportsysteme bekannt. Man unterscheidet dabei zwischen passiven und aktiven lonentransportsystemen. Bei den passiven lonentransportsystemen stehen die eigentlichen Transportmoleküle, die so genannten lonenkanäle, in Wechselwirkung mit separaten Photorezeptor-Komplexen und werden indirekt über diese Photorezeptoren gesteuert. Beispiele für solche mit einem lonenkanal wechselwirkenden Photorezeptor-Komplexe sind die G-Protein aktivierenden Rhodopsine; siehe z.B. Müller, F., Kaupp, U. B., Signaltransduktion in Sehzellen, Naturwissenschaften 85 (1998) 49-6. Da diese bekannten passiven lonentransportsysteme das Zusammenwirken mehrerer Proteine und anderer Komponenten erfordern, ist es problematisch, sie unter Beibehaltung ihrer Funktion, z.B. durch Co-Expression der entsprechenden Proteine in rekombinanten Systemen, in andere Zellen einzuführen.

Beispiele für lichtgesteuerte aktive lonentransportmoleküle in einer Zellmembran sind die Rhodopsine aus Archaeen (Archaebakterien). Ein bekannter Vertreter eines Archaeen-Rhodopsins ist das Bakteriorhodopsin, bestehend aus einer Proteinkomponente, dem Bakterioopsin, und einem über eine Schiffsche Base gebundenen Retinal. Nach Anregung durch Licht geeigneter Wellenlänge transportiert Bakteriorhodopsin Protonen durch die Membran aus dem Zellinneren nach außen. Der lonentransport verläuft in der Regel langsam (< 100 H⁺/ Sekunde) und die Transportrichtung des Bakteriorhodopsins kann nicht frei bestimmt werden. *In vivo* befördert Bakteriorhodopsin Protonen auch gegen einen bestehenden elektrochemischen Gradienten, der sich aus einem pH-Gradienten und einem elektrischem Gradienten ΔΨ zusammensetzt. Eine solche lichtgesteuerte Protonenpumpe stellt ein vergleichsweise einfaches System dar. Bakteriorhodopsin und verwandte Retinalbindende Proteine wie die Chloridpumpe Halorhodopsin wurden bereits heterolog exprimiert und waren im heterogenen Wirt zum lichtgetriebenen aktiven Membrantransport von Ionen, z. B. H⁺ oder Cl⁻, in der Lage.

Für viele Zwecke sind die bekannten Systeme aufgrund ihrer Komplexität bzw. der vorgegebenen Transportrichtung jedoch ungeeignet.

Eine Aufgabe der vorliegenden Erfindung ist es, Mittel und Wege bereitzustellen, die eine schnelle und leichte Messung und/oder Manipulation von intrazellulären lonenkonzentrationen, insbesondere Protonenkonzentrationen, sowie von elektrischen Gradienten über eine Zellmembran erlaubt.

Eine weitere Aufgabe ist es, ein System, das für das Hochdurchsatzscreening von biologischen Molekülen, insbesondere von pH-regulatorischen Membranproteinen und spannungsabhängigen Membranproteinen geeignet ist, bereitzustellen.

Die der Erfindung zu Grunde liegenden Aufgaben werden erfindungsgemäß durch die Verwendung eines biologischen Photorezeptors als direkt lichtgesteuerter lonenkanal zur Veränderung der lonenleitfähigkeit einer Membran mit Hilfe von Licht gemäß dem unabhängigen Anspruch 1 gelöst. Vorteilhafte Weiterbildungen sind Gegenstand der abhängigen Ansprüche.

Die vorliegende Erfindung schafft eine Verwendung eines biologischen Photorezeptors als direkt lichtgesteuerter lonenkanal zur Veränderung der lonenleitfähigkeit einer Membran mit Hilfe von Licht, bei welcher der Photorezeptor ein Apoprotein und ein lichtsensitives Polyen aufweist, wobei das lichtsensitive Polyen kovalent an das Apoprotein gebunden ist, mit dem Apoprotein wechselwirkt und als lichtempfindliches Tor fungiert, bei welcher das Apoprotein ein lonenkanal bildendes Opsin oder ein Derivat oder Fragment eines lonenkanal bildenden Opsins ist und bei welcher durch Belichtung mittels Veränderung der lonenleitfähigkeit der Membran der Tod einer Zelle hervorgerufen werden kann oder hervorgerufen wird.

Der biologische Fotorezeptor kann in einer Zelle, insbesondere in der Membran einer Zelle exprimiert werden.

Der biologische Fotorezeptor kann alternativ oder zusätzlich in der Membran einer Organelle einer Zelle exprimiert werden, insbesondere in der Membran von Mitochondrien einer Zelle oder in der Membran des endoplasmatischen Retikulums einer Zelle.

Der biologische Fotorezeptor kann selektiv exprimiert werden, insbesondere in Bezug auf ein oder mehrere Gewebe, Zelltypen und/oder Organe.

Insbesondere kann Der biologische Fotorezeptor selektiv in Tumorzellen exprimiert werden.

Die Expression kann durch virale Transfektion erfolgen.

Alternativ oder zusätzlich kann auch das Zuführen von Licht selektiv erfolgen.

Die Verwendung ist möglich in einem Verfahren zur Behandlung eines oder mehrerer Zelltypen, eines oder mehrerer Gewebe, eines oder mehrerer Organe, eines oder mehrerer Organismen und/oder des menschlichen Körpers.

Die Verwendung ist möglich in einem Kit für ein Verfahren zur Behandlung eines oder mehrerer Zelltypen, eines oder mehrerer Gewebe, eines oder mehrerer Organe, eines oder mehrerer Organismen und/oder des menschlichen Körpers.

Die Verwendung ist möglich in einem System für ein Verfahren zur Behandlung eines oder mehrerer Zelltypen, eines oder mehrerer Gewebe, eines oder mehrerer Organe, eines oder mehrerer Organismen und/oder des menschlichen Körpers.

Diese und weitere Aspekte der Erfindung werden auch anhand der nachfolgende Erläuterungen verdeutlicht:

Channelrhodopsine1,2 (ChR1, ChR2) sind 7-Transmembranhelixproteine und gehören zur Klasse der Retinalproteine. In der Vergangenheit wurde gezeigt, dass ChR1 und 2 als Protonenkanal (ChR1) bzw. als Kationenkanäle fungieren (ChR2). In der Folge wurde gezeigt, dass ChR2 mit Hilfe verschiedener molekularbiologischer Methoden in Nervenzellen und transgen in die Retina der Maus bzw. in Muskel- und Nervenzellen von C-elegans exprimiert werden kann. Nach Belichtung zeigen alle Zelltypen in den verschiedenen Organismen eine Depolarisation, was zur Auslösung von Aktionspotentialen bzw. in C-elegans zu photophoben Reaktionen und Muskelkontraktionen führt. Letztere können auch auf Grund der gezielten Expression in das endoplasmatische Retikulum u.a. durch Ausschüttung von Ca²⁺ aus den Zellorganellen hervorgerufen werden.

Eine Dauerbelichtung von HEK-Zellen führt, wie gezeigt wurde, zum Absterben, was unter anderem darauf zurückgeführt werden kann, dass eine permanente Depolarisation, Ca²⁺-Influx und Ca²⁺-Ausschütung aus den Zellorganellen erfolgen. Unter den gewählten Bedingungen ist denkbar, dass ChR2 nicht nur in der Plasmamembran, sondern auch in den Zellorganellen (endo- und sarkoplasmatischem Retikulum) exprimiert wird.

Zielgerichtete funktionelle Expression in das endoplasmatische bzw. sarkoplasmatische Retikulum der Zelle führt nach einer Belichtung zu einer massiven Ca²⁺-Ausschüttung aus den Zellorganellen, so dass die Zellen absterben. Analoges gilt für die selektive Expression von ChR1 oder ChR2 in Mitochondrien, so dass die Belichtung der Zelle zum Zusammenbruch des durch die Atmungskette entstandenen Protonengradienten führt, wodurch die ATP Synthese blockiert wird. D.h. ChR1 und ChR2 wirken als lichtgesteuerte Entkoppler der Atmungskette (Blockierung der ATP-Synthese), womit auch hier der Zelltod herbeigeführt würde.

Nimmt man z. T. bereits erfolgreich durchgeführte experimentelle Ansätze zur Hand, so können durch die optische Anregung nach einer Virustransfektion sehr schonend und mit hoher räumlichen Auflösung, z.B. im □m-Bereich einzelne Zellen oder Zellverbände, d.h. Tumore, insbesondere im Gehirn abgetötet werden. Es ist hier keine Immunreaktion nach der Transfektion zu erwarten ist.

Die vorliegende Erfindung löst die Aufgaben also u.a. durch die Verwendung einer neuen Klasse von Photorezeptoren mit bisher unbekannter Funktion, die direkt lichtgesteuerte passive lonentransportsysteme (einen lichtgesteuerten lonenkanal) darstellen. Dieser Photorezeptor umfasst ein Apoprotein, das eine lonenleitfähigkeit vermittelt, und ein kovalent an das Apoprotein gebundenes lichtsensitives Polyen, das mit dem Apoprotein wechselwirkt und als lichtempfindliches Tor fungiert. Überraschenderweise ist es erfindungsgemäß gelungen, die Vorteile eines relativ einfachen direkt lichtgesteuerten Systems mit den Vorteilen eines passiven lonenkanals zu verbinden.

Bei dem Apoprotein handelt es sich um ein Membranprotein mit mindestens 5 Transmembranhelices, das zur Bindung eines lichtsensitiven Polyens fähig ist. Bevorzugt sind Transmembranproteine mit sechs oder sieben Transmembranhelices. Transmembranproteine mit mehr als sieben Helices, beispielsweise acht, neun oder zehn Transmembranhelices, sind von der Erfindung jedoch ebenfalls umfasst. Darüber hinaus umfasst die Erfindung Transmembranproteine, die zusätzlich zu dem Transmembrananteil C- und/oder N-terminale Sequenzen enthalten, wobei die C-terminalen Sequenzen in das Innere des von der Membran umschlossenen Lumens, beispielsweise das Cytoplasma einer Zelle oder das Innere eines Liposomes, hineinreichen können, oder auch an der Membranaußenoberfläche angeordnet sein können. Das gleiche gilt für die gegebenenfalls vorhandenen N-terminalen Sequenzen, die ebenfalls sowohl intraluminal angeordnet sein können als auch auf der äußeren Oberfläche der Membran. Die Länge der C- und/oder N-terminalen Sequenzen unterliegt im Prinzip keiner Beschränkung; bevorzugt sind jedoch Apoproteine mit C-terminalen, nicht in die Membran eingebetteten Sequenzen mit 1 bis 1000 Aminosäuren, bevorzugt 1 bis 500, besonders bevorzugt 5 bis 50 Aminosäuren. Unabhängig von der Länge der C-terminalen Sequenzen umfassen die N-terminal angeordneten, nicht in die Membran eingebetteten Sequenzen bevorzugt 1 bis 500 Aminosäuren, besonders bevorzugt 5 bis 50 Aminosäuren.

Der Begriff der Transmembranhelix ist dem Fachmann bekannt. Generell handelt es sich um α-helicale Proteinstrukturen, die in der Regel 20 bis 25 Aminosäuren umfassen. Je nach Beschaffenheit der Membran, bei der es sich um eine natürliche Membran, beispielsweise eine Zell- oder Plasmamembran, oder auch eine synthetische Membran handeln kann, können die Transmembransegmente jedoch auch kürzer oder länger sein. Beispielsweise können Transmembransegmente in artifiziellen Membranen bis zu 30 Aminosäuren umfassen, andererseits aber auch nur wenige Aminosäuren, zum Beispiel 12 bis 16.

Der erfindungsgemäße lonenkanal kann prinzipiell dem passiven Transport aller physiologisch wichtigen Ionen dienen. Die bekanntesten lonentransportsysteme transportieren Na⁺, K⁺, Ca²⁺, H⁺ oder Cl⁻. In einer bevorzugten Ausführungsform ist der erfindungsgemäße lonenkanal ein Protonenkanal.

In einer besonders bevorzugten Ausführungsform umfasst der Protonenkanal als Apoproteinanteil ein Opsin-Protein oder ein Derivat oder Fragment eines natürlich vorkommenden Opsinproteins. Als Opsine werden hier Apoproteine bezeichnet, die kovalent mit einem Retinoid-Chromophor verbunden sind und lonenleitfähigkeit aufweisen, sobald sie Licht absorbieren. Ein Molekül, das ein kovalent mit einem Opsin verbundenes Retinoid-Chromophor enthält, wird als Rhodopsin bezeichnet.

Ein Derivat eines natürlich vorkommenden und als lichtgeschalteter lonenkanal fungierenden Opsinmoleküles ist dem Original gegenüber durch einen Austausch einer oder mehrerer Aminosäuren, durch eine Insertion und/oder eine Deletion einer oder mehrerer Aminosäuren an einer oder mehreren Positionen verändert. Sowohl die natürlich vorkommenden Opsinmoleküle als auch ihre Derivate sollten im Bereich der 5 Transmembranhelices, die den Helices 3 bis 7 im Bakteriorhodopsin entsprechen, eine Identität von mindestens 15 % mit der Sequenz des Bakteriorhodopsins aufweisen. Eine Identität von 20 % oder mehr zwischen dem Derivat eines Kanal-Opsins und Bakteriorhodopsin, bezogen nur auf den Bereich der Helices 3 bis 7 in Bakteriorhodopsin, ist dabei bevorzugt. Die Identität in den Bereichen des Opsinderivates, die nicht den Helices 3 bis 7 entsprechen, kann dagegen weitaus geringer sein.

Mit dem Begriff "Identität" wird dabei der Grad der Verwandtschaft zwischen zwei oder mehr Proteinsequenzen bezeichnet, der durch die Übereinstimmung zwischen diesen Sequenzen bestimmt wird. Der Prozentsatz der Identität ergibt sich aus dem Prozentsatz identischer Aminosäuren in zwei oder mehr Sequenzen unter Berücksichtigung von Lücken und anderen Sequenzbesonderheiten.

Die Identität miteinander verwandter Protein-Moleküle kann mit Hilfe bekannter Verfahren bestimmt werden. In der Regel werden spezielle Computerprogramme mit den besonderen Anforderungen Rechnung tragenden Algorithmen eingesetzt. Bevorzugte Verfahren zur Bestimmung der Identität erzeugen zunächst die größte Übereinstimmung zwischen den untersuchten Sequenzen. Computerprogramme zur Bestimmung der Identität zwischen zwei Sequenzen umfassen, sind jedoch nicht eingeschränkt auf, das GCG-Programmpaket, einschließlich GAP (Devereux et al., 1984); Genetics Computer Group University of Wisconsin, Madison, (WI)); BLASTP, BLASTN und FASTA (Altschul et al., NCB NLM NIH Bethesda MD 20894; Altschul et al., 1990). Auch der bekannte Smith Waterman-Algorithmus kann zur Bestimmung von Identität verwendet werden.

Bevorzugte Parameter für den Sequenz-Vergleich umfassen die nachstehenden:
Algorithmus: Altschul et al., 1990 (Basic local alignment search tool, J. Mol. Biol. 215, 403-410) (=BLAST)
Vergleichsmatrix: BLOSUM 62 (Henikoff and Henikoff, 1992, Amino acid substitutions from protein blocks. PNAS 89, 10915-10919)
Übereinstimmung (matches) = variabel
Nichtübereinstimmung (mismatch) = variabel
Lücken-Wert: open 10
Lückenlängen-Wert (gap length penalty): 1

Das GAP-Programm ist auch zur Verwendung mit den vorstehenden Parametern geeignet. Die vorstehenden Parameter sind die Standardparameter (default parameters) für Protein-Vergleiche.

Weitere beispielhafte Algorithmen, Lückenöffnungs-Werte (gap opening penalties), Lückenausdehnungs-Werte (gap extension penalties), Vergleichsmatrizen einschließlich der im Programm-Handbuch, Wisconsin-Paket, Version 9, September 1997, genannten, können verwendet werden. Die Auswahl wird von dem durchzuführenden Vergleich abhängen und weiterhin davon, ob der Vergleich zwischen Sequenzpaaren, wobei GAP oder Best Fit bevorzugt sind, oder zwischen einer Sequenz und einer umfangreichen Sequenz-Datenbank, wobei FASTA oder BLAST bevorzugt sind, durchgeführt wird.

Für die aktive Protonenpumpe Bakteriorhodopsin ist bekannt, dass Asp(D)⁹⁶ eine für die Protonenpumpfunktion essentielle Aminosäure ist. Weiter sind die folgenden 16 Aminosäuren des Bakteriorhodopsins am Protonennetzwerk beteiligt:
F⁴², T⁴⁶, Y⁵⁷, R⁸², D⁸⁵, T⁸⁹, L⁹³, T¹⁰⁷, W¹⁸², Y¹⁸⁵, W¹⁸⁹, E¹⁹⁴, E²⁰⁴, D²¹², K²¹⁶, F²¹⁹

Im erfindungsgemäßen lonenkanal ist an der dem D⁹⁶ der Bakteriorhodopsinsequenz entsprechenden Position eine andere Aminosäure als "D". E²⁰⁴ ist vorzugsweise ausgetauscht durch S. In einer Ausführungsform sind von den weiteren 15 Aminosäuren jedoch mindestens 8 identisch erhalten oder lediglich durch konservativen Austausch verändert. Bei den Aminosäuren, die möglichst identisch erhalten sein sollten, handelt es sich bevorzugt um T⁴⁶, Y⁵⁷, R⁸², T⁸⁹, T¹⁰⁷, W¹⁸², E¹⁹⁴, D²¹² und K²¹⁶. Konservativ ausgetauschte Aminosäuren sind bevorzugt F⁴², D⁸⁵, L⁹³, Y¹⁸⁵, W¹⁸⁹ und F²¹⁹ Der Fachmann weiß dabei, dass für einen konservativen Austausch eine Aminosäure ausgewählt wird, die der auszutauschenden Aminosäure funktionell ähnlich ist. Dabei werden Austausche normalerweise innerhalb der folgenden Gruppen vorgenommen:

| | | | | | |
|---|---|---|---|---|---|
| (a) | Ala (A) | Ser (S) | Thr (T) | Pro (P) | Gly (G); |
| (b) | Asn (N) | Asp (D) | Glu (E) | Gln (Q); | |
| (c) | His (H) | Arg (R) | Lys (K) | | |
| (d) | Met (M) | Leu (L) | Ile (I) | Val (V) | und |
| (e) | Phe (F) | Tyr (Y) | Trp (W). | | |

Bezogen auf die oben angegebenen Aminosäuren, sind bevorzugte Austausche die folgenden: F42Y, D85E, Y185F, W189F und F219W.

In einer weiteren bevorzugten Ausführungsform sind ein oder mehrere weitere der folgenden Positionen, bezogen auf Bakteriorhodopsin, im erfindungsgemäßen lonenkanal erhalten: Y⁸³, W⁸⁶, P⁹¹, G¹²², P¹⁸⁶ und G¹⁹⁵_{.}

In einer weiteren bevorzugten Ausführungsform enthält ein erfindungsgemäßer passiver Protonenkanal ein Apoprotein mit der Konsensussequenz L(I)DxxxKxxW(F,Y). In Klammern angegebene Aminosäuren können jeweils die vorhergehende Aminosäure ersetzen. Bei dieser Konsensussequenz handelt es sich um das die Retinalbindende Aminosäure Lysin umgebende Motiv. Im Bakteriorhodopsin entspricht das "K" an Position 6 der Konsensussequenz dem K²¹⁶ in der 7. Helix des Bakteriorhodopsins.

In einer bevorzugten Ausführungsform umfasst der lonenkanal ein Apoprotein aus niederen Eukaryonten. Die Gruppe der niederen Eukaryonten umfasst beispielsweise Algen, Protozoen, Ciliaten und Hefen.

Besonders bevorzugt sind dabei motile Grünalgen, insbesondere Chlorophyceen. Apoproteine aus Volvocales sind dabei besonders interessant. In der am meisten bevorzugten Ausführungsform ist das Apoprotein ein Opsinprotein aus *Chlamydomonas reinhardtii*. Weitere Grünalgen mit bevorzugten Ausführungsformen sind zu finden unter den Ulvophyten wie *Acetabularia* und *Ulva.* Weitere bevorzugte Ausführungsformen sind Opsine aus Prasinophyceen, beispielsweise *Pyramimonas* und *Platymonas (Tetraselmis*). Andere bevorzugte Formen stammen aus dem Königreich der Dinophyten mit der einzigen Klasse der Dinophyceaen und den beispielhaften Vertretern *Gymnodinium splendens, Gyrodinium dorsum, Peridinium balticum* und *Gonyaulax*.

In einer weiteren bevorzugten Ausführungsform stammt das als lichtgesteuerter lonenkanal fungierende Opsin aus einem Protozoon, einem Bakterium oder einem Archaebakterium.

In einer weiteren bevorzugten Ausführungsform stammt das als lichtgesteuerter lonenkanal fungierende Opsin aus Pilzen wie *Neurospora crassa, Fusarium sporotrichioides* and *Leptosphaeria maculans, oder Chytridiomyceten* wie z.B. *Allomyces reticulatus*, oder aus Ciliaten wie *Fabrea salina* oder *Paramecium bursaria* oder aus Foraminiferen wie *Amphistegina radiata*.

Erfindungsgemäss wurden aus *Chlamydomonas reinhardtii* zwei verschiedene Proteine mit bekannter Sequenz funktionell exprimiert und erstmals als passive lonentransportsysteme identifiziert. Dabei handelt es sich um das Channelopsinl (= CHOP-1; auch Chlamyopsin-3 = COP3 genannt) und das Channelopsin2 (= CHOP-2; auch Chlamyopsin-4 =COP4 genannt).

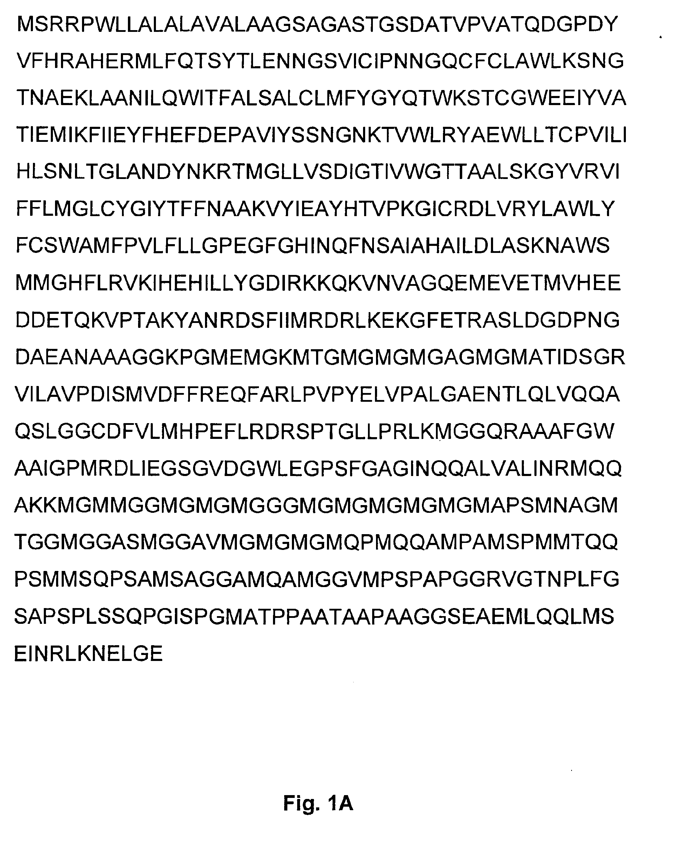

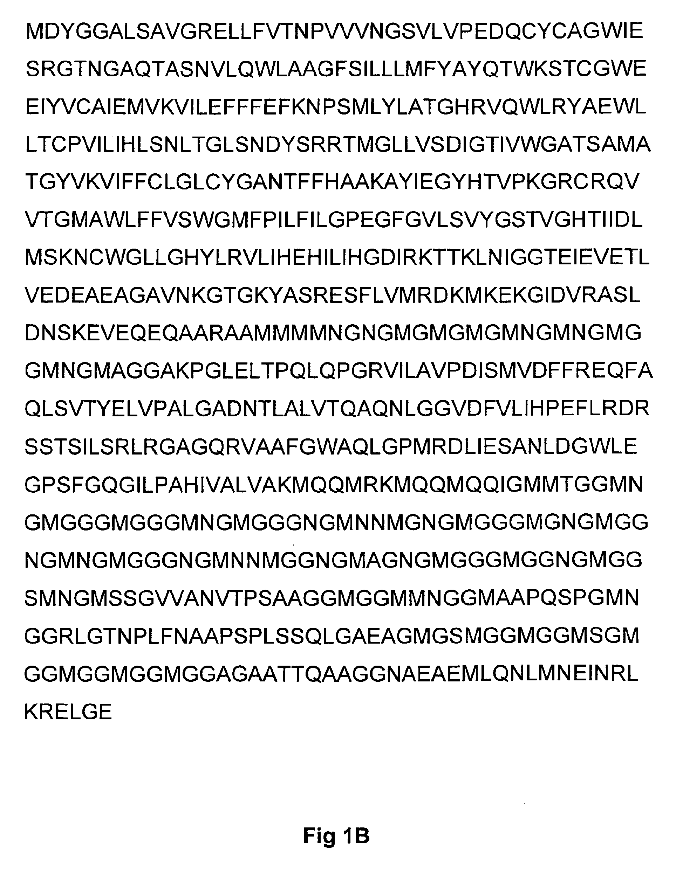

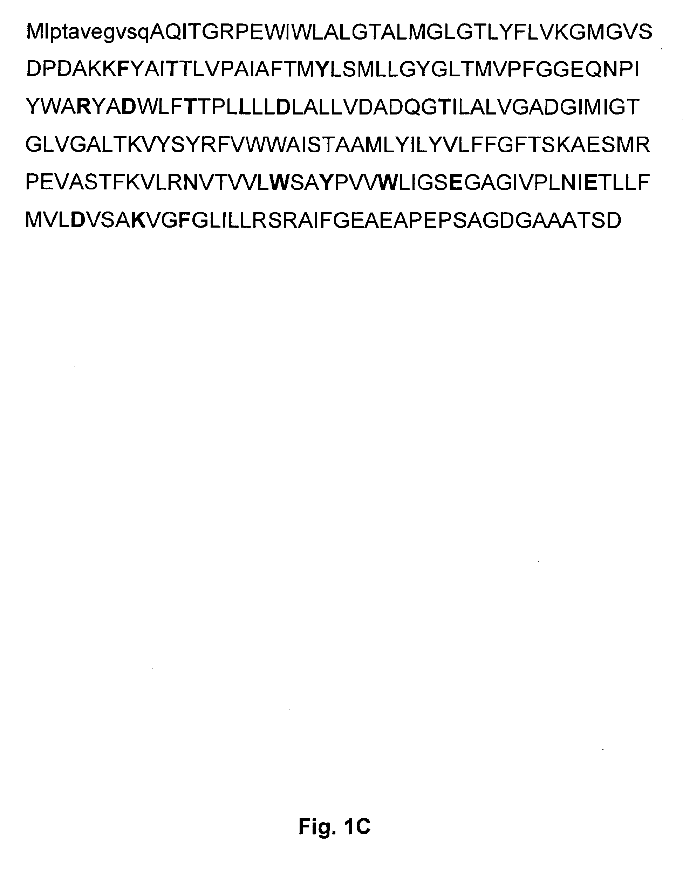

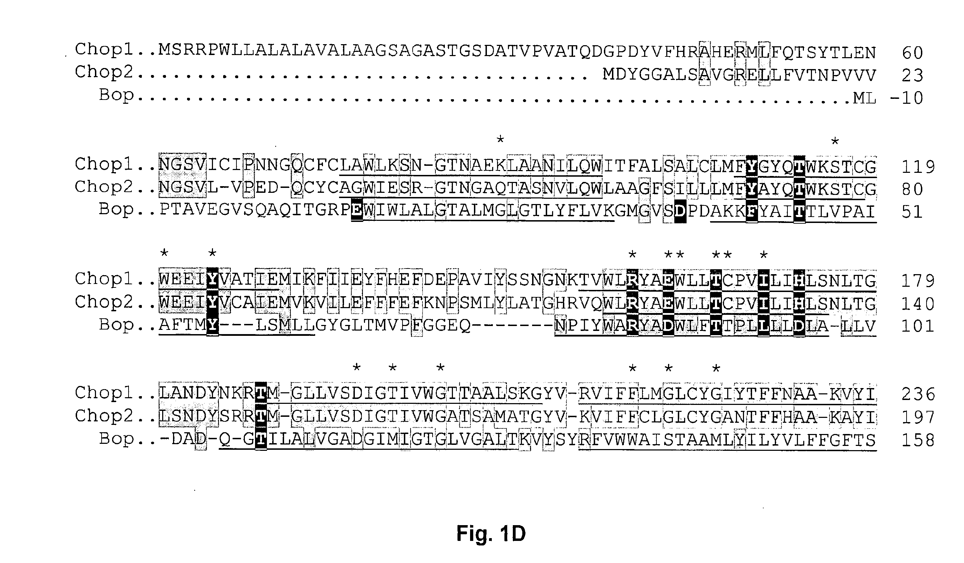

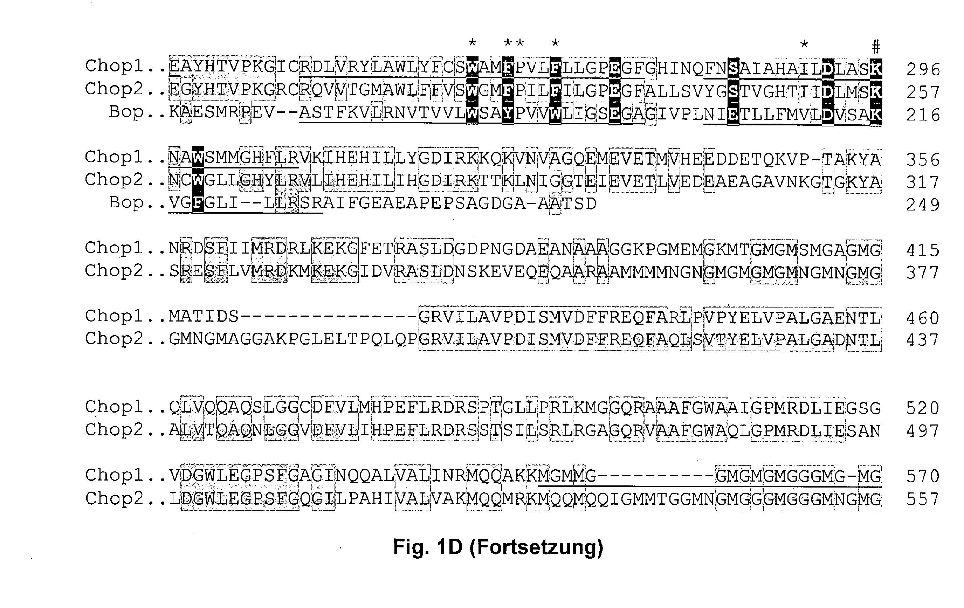

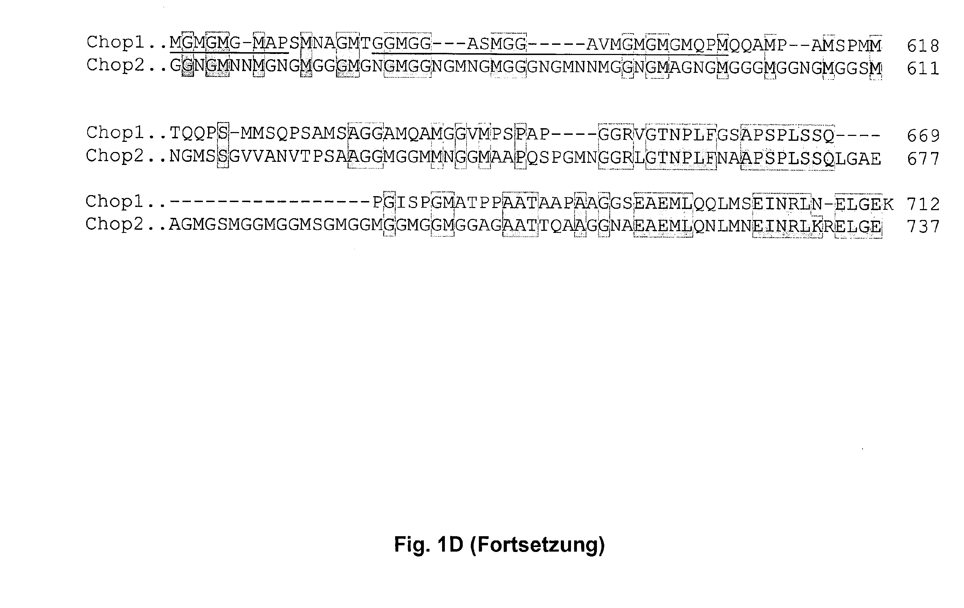

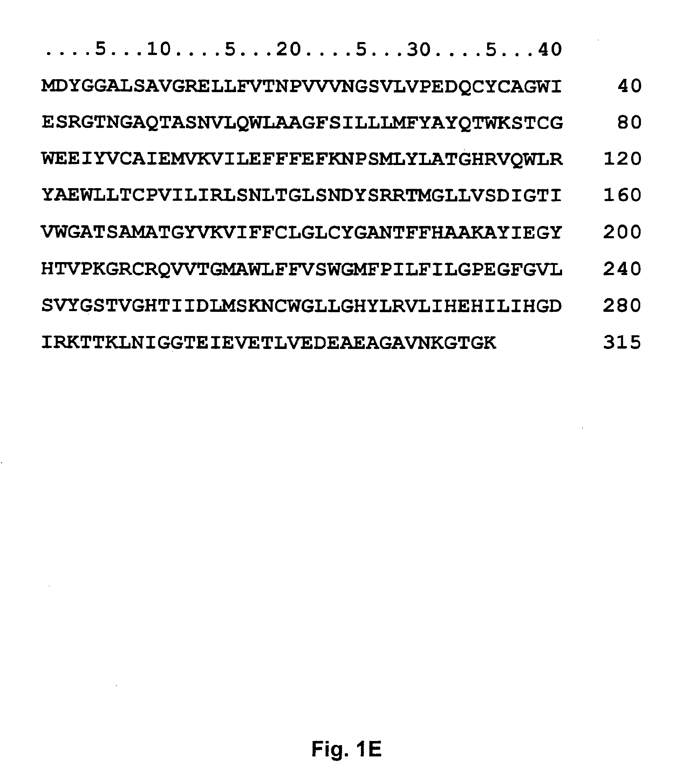

Das CHOP-1-Protein weist ein Molekulargewicht von 76 kD und eine Länge von 712 Aminosäuren auf. Es wurde anhand von überlappenden partiellen cDNA-Sequenzen in einer *C. reinhardtii*-EST-Datenbank (Asamizu et al., DNA Research 7, 305-7 (2000)) identifiziert. Seine Aminosäuresequenz ist in Fig. 1 der vorliegenden Anmeldung dargestellt. Das Core-Protein (Aminosäuren 76-309) umfasst 7 hypothetische Transmembran-Segmente mit 15-20% Homologie zu den sensorischen Archaeen-Rhodopsinen, den lonentransportern Bakteriorhodopsin (BR) und Halorhodopsin (HR), sowie zu einem erst kürzlich identifizierten Rhodopsin aus dem Pilz *Neurospora crassa* (NOP1). Diese Homologiegrade sind zwar quantitativ relativ gering, es sind jedoch im Vergleich zu BR speziell diejenigen Aminosäuren konserviert, welche die Retinal-Bindungsstelle und das H⁺-Transport-Netzwerk in BR definieren. Das beobachtete Konsensus-Motiv LDxxxKxxW legt nahe, dass im CHOP-1 K²⁹⁶ die Retinalbindende Aminosäure ist. 9 von 22 Aminosäuren, welche im Bakteriorhodopsin in direktem Kontakt mit dem Retinal stehen, sind in CHOP-1 identisch erhalten und 4 weitere reflektieren lediglich konservative Änderungen ((Fig.1); Nagel et al., in Vorbereitung).

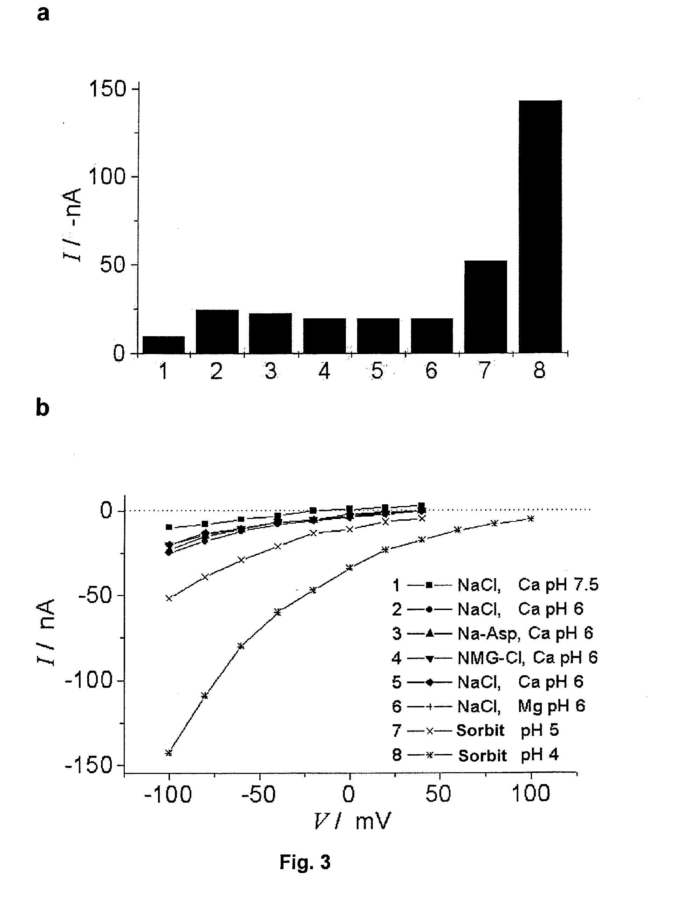

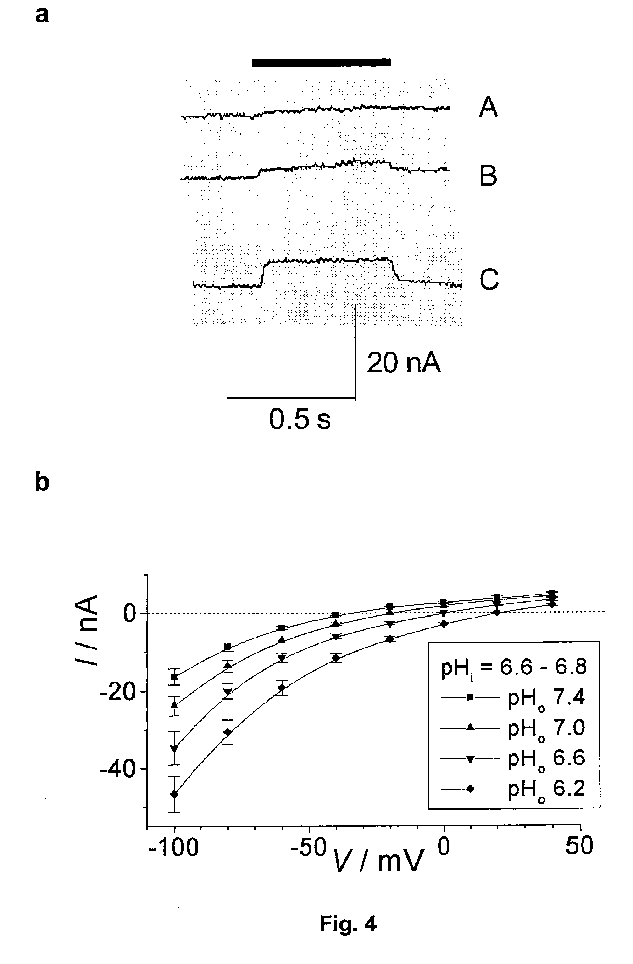

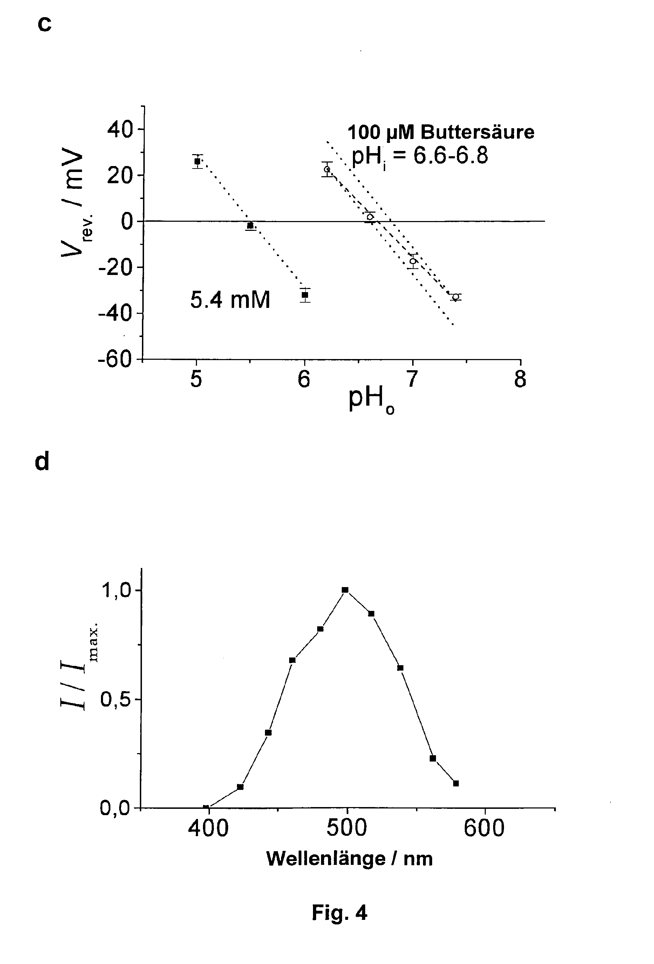

Eingehende Untersuchungen der lichtgesteuerten lonentransportfunktion des CHOP-1-Proteins in Oozyten von *Xenopus laevis* zeigten, dass es sich bei den transportierten Ionen um Protonen handelt (Fig. 3), außerdem, dass der lonentransport rein passiver Natur ist (Fig. 4a-c). Der induzierte Photostrom und damit der lonentransport ist von der Wellenlänge des Anregungslichtes abhängig und erreicht ein Maximum bei 500 nm (Fig. 4d).

Analoge Versuche mit zwei kürzeren Fragmenten des CHOP-1-Proteins, welche die Aminosäuren 1-346 bzw. 1-517 umfassten, lieferten Ergebnisse, welche mit denjenigen des CHOP-1-Proteins vollständiger Länge im Wesentlichen identisch waren. Dies demonstriert, dass ein großer Teil des carboxyterminalen Bereichs des CHOP-1-Proteins für die lonentransportfunktion nicht erforderlich ist.

Das CHOP-1-Protein aus *C. reinhardtii* stellt den ersten identifizierten Vertreter eines neuen direkt lichtgesteuerten passiven lonentransportproteins dar. Strukturell und/oder funktionell ähnliche Rhodopsin-Proteine kommen auch in anderen Mikroalgen sowie in Gameten und Zoosporen von Makroalgen und möglicherweise auch in anderen Organismen vor.

Das zweite als Apoprotein eines lichtgeschalteten lonenkanals identifizierte Protein ist Channelopsin2 (CHOP-2), dessen 737 Aminosäuren umfassende Sequenz ebenfalls in Figur 1 gezeigt ist. Es zeigt eine Homologie von 52.7 % zu CHOP-1. Die über die Homologie zwischen BR und CHOP-1 sowie Modellberechnungen identifizierten, für den Transport wichtigen Aminosäuren sind auch in CHOP-2 weitgehend konserviert. Auch für dieses Rhodopsin wurde eine lichtgeschaltete passive Protonenleitfähigkeit durch Expression in *Xenopus*-Oocyten erstmalig nachgewiesen. Der mit CHOP-2 als Apoprotein gebildete lonenkanal unterscheidet sich von dem mit CHOP-1 gebildeten hinsichtlich seiner Einheitsleitfähigkeit, seiner Inaktivierung im Dauerlicht und der Form der Strom-Spannungskurve. Channelrhodopsin-2 (ChR2), welches sich aus dem Protein Channelopsin-2 (CHOP-2) und Retinal zusammensetzt, ist ein lichtgesteuerter Kationenkanal, der z.B. für Li⁺, Na⁺, K⁺, Ba²⁺, Sr⁺ und Ca²⁺, nicht jedoch für Mg²⁺, permeabel ist. Das Maximum des Anregungslichts liegt bei 440-480 nm.

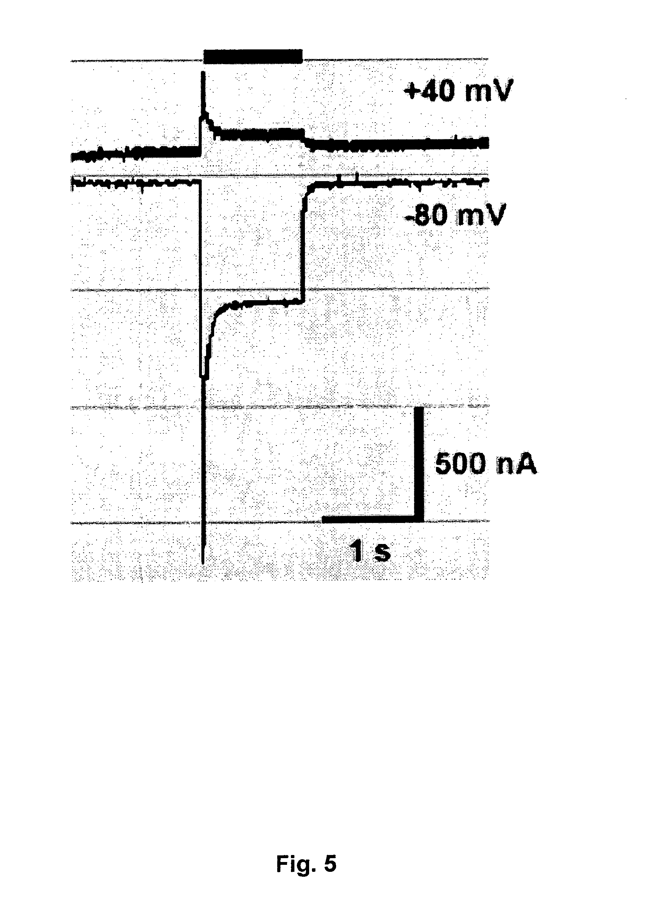

Ähnlich wie bei dem CHOP-1 Protein, konnte auch hier gezeigt werden, dass eine verkürzte Form von CHOP-2, welche die Aminosäuren 1-315 umfasst, ebenfalls einen funktionsfähigen lichtgeschalteten lonenkanal bildet (Fig. 5-7).

CHOP-2 und oder CHOP-2-315 können zur Depolarisation von Zellen (siehe Fig. 7) oder zur Ca²⁺-Aufnahme (Fig. 6) oder zu beidem benutzt werden.

Im Rahmen der vorliegenden Erfindung ist der Fachmann in der Lage, Derivate der ursprünglichen Aminosäuresequenz in Kenntnis der Nukleotid- und Aminosäuresäuresequenz sowie der berechneten 3D-Struktur des natürlichen CHOP-1- und CHOP-2-Proteins, durch Austausch und/oder Insertion und/oder Deletion von einer oder mehreren Aminosäure(n) herzustellen, welche die lonentransporteigenschaften des ursprünglichen Proteins ganz oder teilweise behalten haben oder sich bezüglich lonenspezifität, Inaktivierung, Gleichrichtung etc. signifikant unterscheiden. Derivate sind dabei wie oben angegeben definiert.

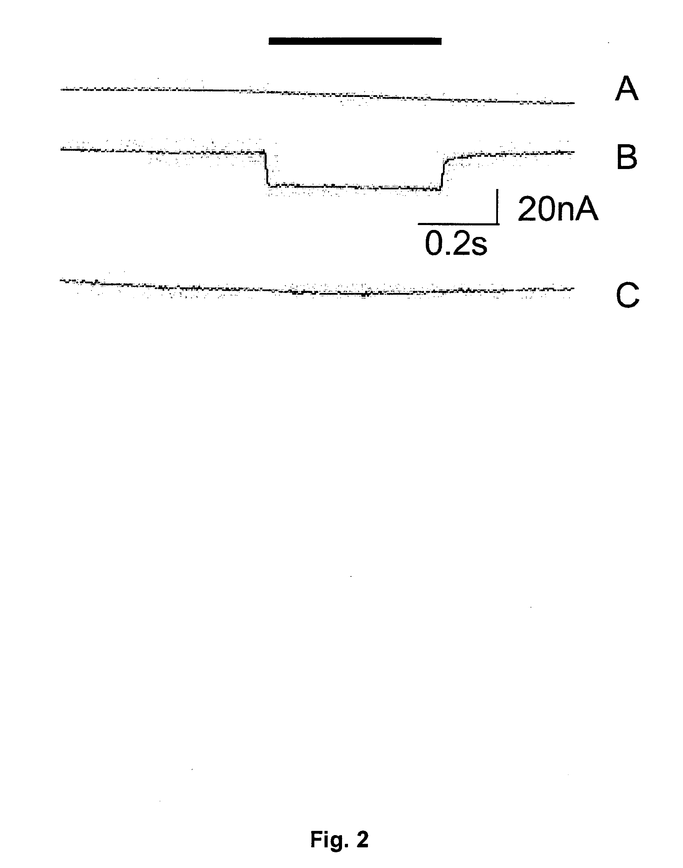

Aktive lonentransportsysteme können von passiven Systemen elektrophysiologisch eindeutig und zweifelsfrei unterschieden werden, wie nachfolgend ausgeführt. Passive lichtgesteuerte lonenkanäle, die Gegenstand der Erfindung sind, erhöhen im Licht die Leitfähigkeit von Membranen, in die sie eingebaut sind, gegenüber dem Dunkelzustand. Die lonenströme, die bei Belichtung auftreten (Photoströme), werden von der lonenspezies getragen, die von dem lichtgesteuerten lonenkanal geleitet wird. Bei gleicher Konzentration der geleiteten Ionen (bei CHOP-1 Protonen; bei CHOP-2 z.B. Protonen) auf beiden Seiten der Membran (symmetrische Bedingungen) wird die Richtung des Photostroms allein durch das angelegte Membranpotential bestimmt. Im Falle eines positiven Ladungsträgers erfolgt bei negativem Potential (in der Zelle negativ) ein Einwärtsstrom unter Bewegung der positiv geladenen Ionen in die Zelle, bei positivem Potential (innen positiv) ein Auswärtsstrom unter Bewegung der positiv geladenen Ionen aus der Zelle heraus. Die Strom-Spannungskurven eines erfindungsgemäßen lichtgesteuerten lonenkanals gehen bei symmetrischer Verteilung des transportierten Ions immer durch den Nullpunkt (Umkehrpotential E_{H}=0). Die Strom-Spannungskurven der erfindungsgemäßen lichtgesteuerten lonenkanäle sind jedoch nicht notwendigerweise linear. In der bevorzugten Ausführungsform von CHOP-1 zum Beispiel wird der Strom in Einwärtsrichtung besser geleitet als in Auswärtsrichtung (Einwärtsgleichrichter, siehe Fig. 2 und 3).

Im Gegensatz dazu geht bei allen aktiven Transportsystemen (wie z. B. Bakteriorhodopsin) die Strom-Spannungskurve bei symmetrischer lonenverteilung nie durch Null. Die Einstellung einer Ungleichverteilung der geleiteten Ionen zwischen innen und außen hat eine Verschiebung der Strom-Spannungskurven entlang der Spannungsskala entsprechend der Nernst'schen Gleichung zur Folge, d.h., um 58 mV bei monovalenten Ionen, um 29 mV bei divalenten Ionen.

Durch Untersuchung der Strom-Spannungskurven kann daher für jeden lichtgesteuerten lonenkanal festgestellt werden, ob es sich um ein passives oder aktives System handelt.

Die vorliegende Erfindung betrifft daher auch Derivate und Fragmente der CHOP-Proteine, welche die lonentransporteigenschaften des ursprünglichen Proteins ganz oder teilweise behalten haben oder sogar in gesteigertem Maße aufweisen, sowie strukturell und funktionell ähnliche Opsine aus anderen Organismen, natürlicher Herkunft oder durch Anwendung rekombinanter Techniken modifiziert, wenn sie die genannten biophysikalischen Eigenschaften aufweisen und wie CHOP-1 und CHOP-2 zu den genannten Zwecken eingesetzt werden können.

Umfangreiche Versuchsreihen zur Herstellung und Untersuchung solcher Derivate führten zu dem überraschenden Ergebnis, dass die Aminosäureposition 134 im CHOP-2-Protein für die Funktion als lichtgeschalteter lonenkanal von besonderer Bedeutung ist. So kann beispielsweise durch Austausch von Histidin an Position 134 gegen Arginin eine besonders aktive Mutante des vollständigen CHOP-2-Proteins bzw. des verkürzten CHOP-2-315-Proteins erzeugt werden. Dieser Austausch kann nach Standardverfahren z. B. durch stellengerichtete Oligonukleotid-Mutagenese durchgeführt werden. Weitere Daten legen nahe, dass Glu123 an einer lichtinduzierten Deprotonierung beteiligt ist, die zu einer Desensibilisierung des lonenkanals führt. So erhöhte die Mutation von Glu123 zu Asp (E123D) das Verhältnis des transienten Photostroms zu dem Gleichgewichts-Photostrom deutlich, während die Mutation von Glu123 zu Gln (E123Q) den Peak des transienten Photostroms fast verschwinden lässt, aber auch zu einer starken Verringerung des stationären Photostroms führt.

Das erfindungsgemäße passive lonentransportsystem enthält ein lichtsensitives Polyen. Dabei kann es sich beispielsweise um p-Hydroxyzimtsäure, Retinal oder ein Retinalderivat handeln. Bevorzugte Retinalderivate sind aus der folgenden Gruppe ausgewählt: 3,4-Dehydroretinal, 13-Ethylretinal, 9-dm-Retinal, 3-Hydroxyretinal, 4-Hydroxyretinal, Naphthylretinal; 3,7,11-Trimethyl-dodeca-2,4,6,8,10-Pentaenal; 3,7-Dimethyl-deca-2,4,6,8-tetraenal; 3,7-Dimethyl-octa-2,4,6-trienal; sowie 6-7-, oder 10-11-rotations-blockierte Retinale mit 4-, 5-, 6- oder 7-Ringverbrückungen. Besonders bevorzugt ist jedoch ein 10-12-Fünfring-verbrücktes Retinal (Takahashi et al. FEBS Lett. 314, 275-279).

Nach Lichtabsorption und Isomerisierung der Polyene kommt es in den erfindungsgemäßen lichtgesteuerten lonenkanälen zu einer strukturellen Änderung im Protein (Opsin) und damit zur Öffnung des Ionen leitenden Kanals, der die intrazelluläre Seite der Membran mit der extrazellulären verbindet. Dieses Ereignis unterscheidet sich grundsätzlich von der Situation in den bekannten lonenpumpen, bei denen der extrazelluläre protonenleitende Halbkanal (EC) mit dem intrazellulären Halbkanal (IC) nie leitend verbunden ist. Im Bakteriorhodopsin ist die Schiffsche Base des Retinal im frühen M-Intermediat des Reaktionszyklus (Photozyklus) mit der extrazellulären Seite leitend verbunden, im späten M-Zustand dagegen ist sie mit der intrazellulären Seite verbunden.

Die erfindungsgemäßen lichtgesteuerten lonenkanäle können in eine Membran, beispielsweise die Plasmamembran einer Zelle, eingebaut und dazu verwendet werden, das Membranpotential durch Belichtung schnell und definiert zu verändern, was zur Aufklärung der Mechanismen von spannungsabhängigen lonenkanälen oder lonentransportern sehr hilfreich ist. Ferner ergibt sich so die Möglichkeit, mit einem lichtgesteuerten gezielten lonentransport die intrazellulären Niveaus dieser Ionen schnell und definiert zu verändern.

Im Falle des CHOP-1-Proteins bedeutet dies eine gezielte nicht invasive Veränderung des intrazellulären pH-Werts, was zur Aufklärung der Mechanismen der intrazellulären pH-Regulation bzw. des Einflusses von transienten pH-Änderungen auf zelleigene Proteine von Nutzen ist.

Durch die Messung des Umkehrpotentials der lichtinduzierten CHOP-1-vermittelten Protonenleitfähigkeit kann der intrazelluläre pH-Wert direkt unterhalb der Membran, bzw. ein pH-Gradient über die Membran bei Kenntnis des extrazellulären pHs schnell und genau gemessen werden. Hierzu wird mit der in Fig. 2-4 angewandten "Voltage Clamp" Messung das Umkehrpotential bestimmt und damit die Zellen geeicht. Nachfolgend kann die Modulation des Membranpotentials und/oder des intrazellulären pH-Werts mit Licht über den erfindungsgemäßen lichtgesteuerten lonenkanal nichtinvasiv erfolgen. Die entsprechenden Messungen erfolgen schnell und definiert und eignen sich so in idealer Weise für moderne HTS (High-Throughput-Screening)-Geräte, mit denen sich beispielsweise pH-regulierte spannungsabhängige Membranproteine wie lonenkanäle in Screeninganwendungen mit hohem Durchsatz testen lassen. Spannungs- oder pH-Sprünge lassen sich in Zelllinien, die ChR1 oder verwandte Rhodopsine enthalten, mit Licht induzieren.

Eine besonders interessante Anwendung ist die optoelektrische Kopplung durch lichtgesteuerte Modulation des Membranpotentials. Diese Anwendung beruht auf einer Kombination des lichtgesteuerten passiven lonenkanals mit einem lichtgesteuerten aktiven lonentransportsystem, z.B. einer lonenpumpe, wobei zur Lichtsteuerung des passiven lonenkanals vorzugsweise eine andere Wellenlänge als zur Lichtsteuerung des aktiven lonentransportsystems eingesetzt wird. In einer bevorzugten Ausführungsform wird Licht einer Wellenlänge von 650 nm zur Aktivierung der Protonenpumpe Bakteriorhodopsin und zum Aufbau eines Membranpotentials eingesetzt und anschließend Licht einer Wellenlänge von 440 nm, das auf Bakteriorhodopsin inhibitorisch wirkt, zur Aktivierung des CHOP-1-Protonentransportsystems und zur schnellen Dissipation des Potentials eingesetzt.

Lichtregulierte lonenkanäle können auch bei der Signalübertragung von neuronalen Netzwerken auf Mikroelektrodennetze eingesetzt werden. Hierbei wird versucht, elektrische Impulse von Neuronen auf Mikrocomputer zu übertragen ("Interfacing von Nervenzellen und Halbleitern": Fromherz (2001), Physikalische Blätter 57, 43-48). Bislang mussten die Neuronen entweder über Neurotransmitter oder direkt mit Mikropipetten stimuliert werden. Neuronen, die ChR1, ChR2 oder verwandte lichtgesteuerte lonenkanäle exprimieren, könnten über Licht gesteuert werden.

Eine weitere Anwendung ist die Therapie von blinden Tieren oder letztendlich Menschen. Es gibt einige Krankheiten, bei denen die natürlichen Sehzellen nicht mehr funktionieren, aber alle Nervenverbindungen weiterhin arbeiten können. Heute wird in verschiedenen Forschungszentren versucht, dünne Filme mit künstlichen keramischen Photozellen auf der Netzhaut zu implantieren (E. Zrenner (2002)_Science 295, 1022-1025.) Diese Photozellen sollen dazu dienen, die sekundären noch intakten Zellen der Retina zu depolarisieren und damit einen Nervenimpuls auszulösen (*bionische Augen*). Die gezielte Expression von lichtgesteuerten Rhodopsinen wie ChR1 oder ChR2 in diesen Ganglionzellen, Amacrinzellen oder bipolaren Zellen würde eine sehr viel elegantere Lösung darstellen und eine höhere räumliche visuelle Auflösung ermöglichen.

Der Einbau des erfindungsgemäßen Rhodopsin-lonentransportsystems in die Membran von Zellen, welche das entsprechende Opsin-Protein in der Natur nicht exprimieren, kann z.B. in einfacher Weise dadurch erfolgen, dass die für dieses Opsin kodierende DNA unter Anwendung bekannter Verfahren der rekombinanten DNA-Technologie zuerst in einen geeigneten Expressionsvektor, z. B. ein Plasmid, ein Cosmid oder ein Virus, eingebaut wird, damit anschließend die Zielzellen transformiert werden und das Protein in diesem Wirt exprimiert wird. Anschließend werden die Zellen in geeigneter Weise z.B. mit Retinal behandelt, um die Verbindung einer Schiffschen Base zwischen Protein und Retinal zu ermöglichen.

In einer bevorzugten Ausführungsform geschieht dieses in verschiedenen Hefen wie *Saccharomyces cerevisiae, Schizosaccharomyces pombe, Pichia pastoris* wie bereits für aktive Rhodopsine wie Bakteriorhodopsin und/oder Rinderrhodopsin erfolgreich durchgeführt (Hildebrandt V. et al. (1989)), Genetic transfer of the pigment bacteriorhodopsin into the eukaryote Schizosaccharomyces pombe, FEBS Lett. 243(2):137-40; Najmoutin G. et al., Heterologous expression of bovine opsins in Pichia pastoris, Meth. Enzymol. (2000) 315, 3-11).

Die Expression kann auch in bestimmten Säugerzellsystemen erfolgen. Dieses geschieht entweder mit episomalen Vektoren als transiente Expression, vozugsweise in COS-Zellen (generiert durch Infektion von "African green monkey kidney CV1" Zellen) (Oprian et al. (1987) Proc Natl. Acad. Sci USA 84, 8874 ff.) oder HEK-Zellen ("*human embyonic kidney* cells", z.B. HEK293-Zellen, Reeves et al. (1996) Proc. Natl. Acad. Sci USA 84, 11487 ff.) oder BHK-Zellen ("baby hamster kidney cells"), oder in Form einer stabilen Expression (durch Integration ins Genom) in CHO-Zellen ("chinese hamster ovary cells"), Myelom-Zellen oder MDCK-Zellen ("Madine-Darby canine kidney cells") (Übersicht in: Makrides SC (1999) Prot. Expr. Purif. 17, 183-202) oder in durch Baculoviren infizierte Sf9-Insektenzellen (Jansen et al. (1988) Mol Biol. Rep. 13, 65 ff.).

Zur Gewährleistung oder Optimierung der Expression kann die kodierende DNA auch in geeigneter Weise modifiziert werden, beispielsweise durch Verknüpfung mit geeigneten regulatorischen Sequenzen und/oder durch Anpassung der kodierenden DNA-Sequenz an die bevorzugte Codonverwendung des gewählten Expressionssystems.

### Figurenbeschreibung:

Fig. 1
Fig. 1A: Aminosäuresequenz des Channelopsinl (Chop1) SEQ ID NO: AF385748
Fig. 1B: Aminosäuresequenz des Channelopsin2 (Chop2) SEQ ID NO: AF461397
Fig. 1C: Aminosäuresequenz des Bacterioopsins (Bop) aus *Halobacterium salinarium* (BR). Mit kleinen Buchstaben wird die *Leadersequenz* angezeigt, die *in vivo* abgespalten wird und aus historischen Gründen bei der Nummerierung der Aminosäuren nicht mitgezählt wird. In fetten Buchstaben sind die für die Protonenleitung essentiellen Aminosäuren gezeigt.
Fig. 1D: Vergleich der Aminosäuresequenzen von CHOP-1 (SEQ ID NO: AF385748) und CHOP-2 (SEQ ID NO: AF461397) aus *Chlamydomonas reinhardtii* mit der von Bakteriorhodopsin aus *Halobacterium salinarum*. Aminosäuren, von denen für BR (Lücke et al. (1999) Science 286, 255-260 und darin erwähnte Literatur) bekannt ist, dass sie direkt mit dem Retinal wechselwirken, sind durch Sterne angezeigt. Aminosäurepositionen, die in mindestens zwei Sequenzen gleich sind, sind hellgrau unterlegt. Aminosäuren, die zu dem H⁺-leitenden Netzwerk in BR beitragen und die diesen entsprechenden Aminosäuren in den anderen Opsinen sind weiß auf schwarzem Grund. Für das His 173 von CHOP-1 wurde im Rahmen der Erfindung gezeigt, dass es an der Protonenleitung beteiligt ist. # indiziert die Position des Retinal-bindenden Lysins. Die unterstrichenen Aminosäuren zeigen die 7 Transmembran-Helices des Core-Proteins an.
Fig. 1E: Aminosäuresequenz der CHOP-2-Core-Protein-Mutante CHOP2-315/- H134R), bei der Histidin an Position 134 durch Arginin ersetzt ist.
Fig. 2 Photoströme, die während der Belichtung von Oozyten mit grünem oder rotem Licht (500 ± 25 nm bzw. 700 ± 25 nm, 10²² Photonen, m⁻² s⁻¹) registriert wurden. Haltepotential (Vₕ) = -100 mV, Lichtpuls durch den Balken angezeigt. Badlösung = 96 mM NaCl, 5 mM KCI, 2 mM CaCl₂, 1 mM MgCl₂, 5 mM MOPS, pH 7,5. A) Eine Oozyte, der keine CHOP-1-RNA injiziert wurde, bestrahlt mit grünem Licht B) eine CHOP-1-Oozyte, bestrahlt mit grünem Licht; C) dieselbe Oozyte wie in B), bestrahlt mit rotem Licht.
Fig. 3 Abhängigkeit des lichtinduzierten Einwärtsstroms von den lonenbedingungen und der Spannung.
a: Ergebnisse mit einer (von fünf) charakteristischen CHOP-1-Oozyte, in der Reihenfolge der Messung (etwa 150 s Intervall), dargestellt, Vₕ = -100 mV, grünes Licht wie in Fig. 2. Die Lösungen sind mit 5 mM MOPS (pH 7,5) oder MES (pH 6) oder Citrat (pH 5 und 4) gepuffert. Konzentration in mM:

| | |
|---|---|
| 1. 100 NaCl, 2 CaCl₂, pH 7,5 | 2: 100 NaCl, 2 CaCl₂, pH 6,0 (Referenz) |
| 3: 100 Na-Aspartat, 2 CaCl₂, pH 6,0 | 4: 100 NMG-Cl, 2 CaCl₂, pH 6,0 |
| 5: wie 2 | 6: 100 NaCl, 2 EGTA, 2 MgCl₂, pH 6,0 |
| 7: 200 Sorbit, 5 EGTA, pH 5,0 | 8: 200 Sorbit, 5 EGTA, pH 4,0 |

b: Strom-Spannungs-Verhältnisse der Photoströme von Fig. 3a, Konzentrationen siehe oben.
Fig. 4 Photoströme, die unter Variation des äußeren und inneren pH-Werts aufgezeichnet wurden, und deren Abhängigkeit von der Wellenlänge
a: Photostrom bei Vₕ = +40 mV. Ergebnisse von einer (von fünf) charakteristischen CHOP-1-Oozyten in einer Badlösung von NaCl (100 mM, Spur A: rotes Licht; Spur B: grünes Licht; 10²² Photonen m⁻² s⁻¹) oder Natriumbutyrat (40 mM, +60 mM NaCl, Spur C: grünes Licht) von pH 7,4
b: Strom-Spannungs-Verhältnisse von Photoströmen in verschiedenen Badlösungen, die immer 100 µM undissoziierte Buttersäure enthielten:
   ■, 60 mM NaCl + 40 mM Na-Butyrat, pH 7,4; ▲, 84 mM NaCl + 16 mM Na-Butyrat, pH 7,0; ▼, 93,6 mM NaCl + 6,4 mM Na-Butyrat, pH 6,6; ◆, 97,4 mM NaCl + 2,6 mM Na-Butyrat, pH 6,2;
c: pH-Abhängigkeit von Umkehrpotentialen aus b. Die punktierten Linien zeigen die theoretische Beziehung für einen konstanten pHᵢ von 6,6 bzw. 6,8 und -58 mV/pH. Die gestrichelte Linie zeigt die erwartete Beziehung für eine Steigung von -48 mV/pH-Differenz und pHᵢ = 6,8 bei pHₒ = 7,4. Die Steigung von -48 mV/pH entspricht einem langsam abnehmenden pHᵢ (um 0,17 Einheiten pro pHₒ-Abnahme von einer Einheit). □, pH-Abhängigkeit von Umkehrpotentialen aus Versuchen mit 5,4 mM undissoziierter Essigsäure (n =3). Die punktierte Linie zeigt die theoretische Beziehung für einen konstanten pHᵢ von 5,5 und -58 mV/pH.
d: Wellenlängenabhängigkeit des lichtinduzierten Einstromes bei pHₒ 5,5 und - 40 mV. Die Photoströme wurden für einen gleichen Photonenstrom normiert.

- Fig. 5: Photoströme, die bei der Belichtung (Wellenlänge 450 ± 25 nm) von CHOP2-315 exprimierenden Oozyten in Ringerscher Lösung (110 mM NaCl, 5 mM KCI, 2 mM CaCl₂, 1mM MgCl₂, 5 mM MOPS, pH 7,6) bei +40 mV und -80 mV registriert wurden.

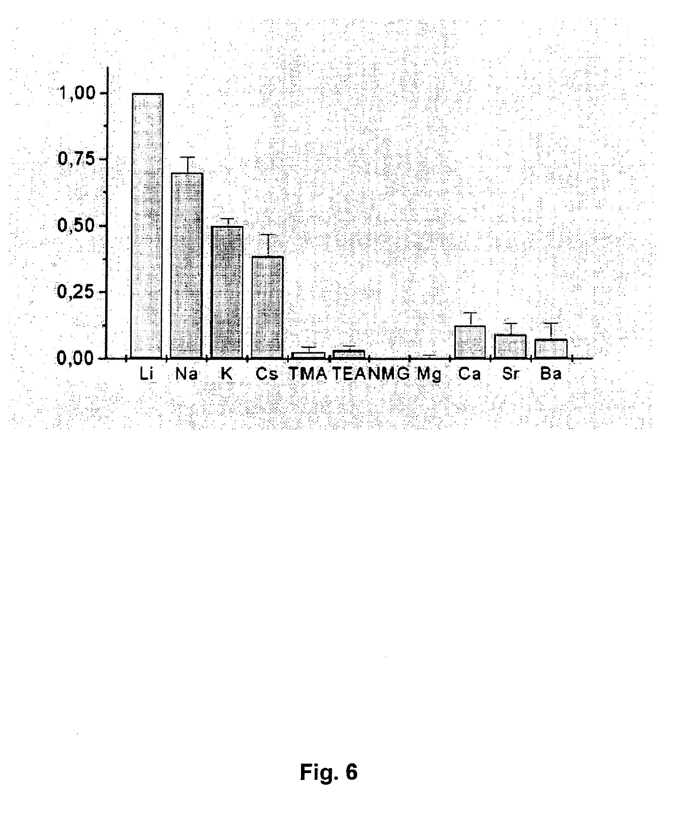

- Fig. 6: Größe des lichtinduzierten Gleichgewichts-Einstroms bei -100 mV, pH 9, für 115 mM Li, Na, Cs, TMA, TEA oder NMG und für 80 mM Mg²⁺, Ca²⁺, Sr²⁺ oder Ba²⁺ (für beide Varianten CHOP2 und CHOP2-315 identisch)

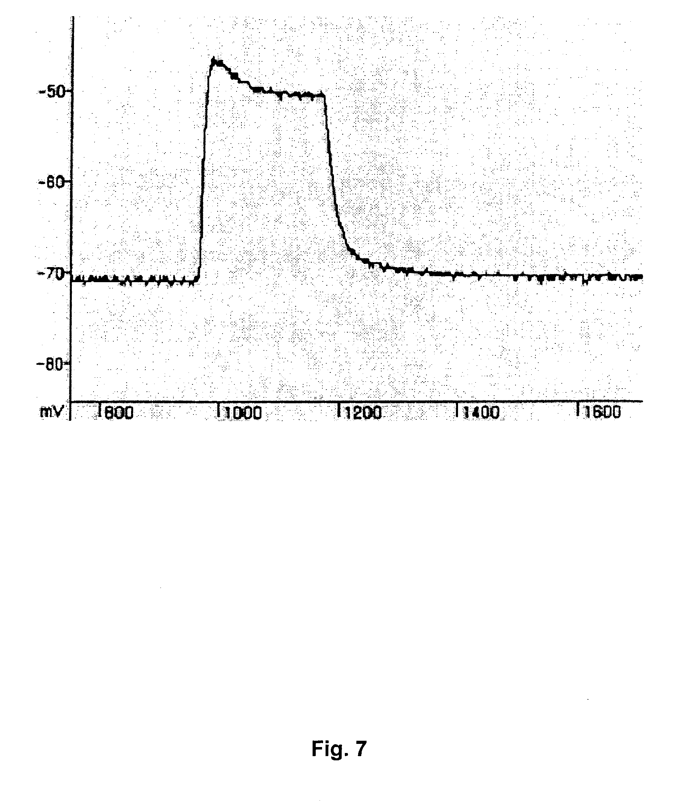

- Fig. 7: Depolarisation einer Oozyten-Plasmamembran, die CHOP2-315 exprimiert, durch einen Lichtblitz von 200 ms

Die folgenden Beispiele erläutern die vorliegende Erfindung, ohne diese jedoch darauf zu beschränken.

### BEISPIEL I

### Amplifizierung von CHOP-1-DNAs und Expression von funktionsfähigen CHOP-1-Photorezeptoren in Xenopus laevis

Eine CHOP-1-DNA vollständiger Länge, kodierend für die Aminosäuren 1-712, und zwei kürzere CHOP-1-DNAs, kodierend für die Aminosäuren 1-346 bzw. 1-517 von CHOP-1, wurden von einer cDNA-Matrize vollständiger Länge mittels PCR unter Verwendung einer Polymerase mit Korrekturfunktion (pfu, Promega) und zwei Startoligonukleotiden (Primern), die BamH1- und HindIII-Restriktionsstellen enthielten, amplifiziert. Die Produkte wurden in den Vektor pGEMHE (Liman et al., Neuron 9, 861-71 (1992)) inseriert und in *E. coli* kloniert. Nach Überprüfung der Sequenzen wurden die CHOP-1-DNAs *in vitro* transkribiert (Ambion) und 20 bis 30 ng cRNA in Oozyten von *Xenopus laevis* injiziert, wie bereits für Bakteriorhodopsin beschrieben (Nagel et al., FEBS Letters, 377, 263-266 (1995). Die Oozyten, die das CHOP-1-Protein exprimierten, wurden mit einem *all-trans*-Retinal (1 µM) in Ringerscher Lösung zwei bis fünf Tage lang inkubiert.

### BEISPIEL II

### Charakterisierung des CHOP-1-Photorezeptors

Zur Untersuchung der angenommenen lonentransportfunktion wurden die CHOP-1-exprimierenden Oozyten unter Anwendung einer Zweielektroden-Spannungsklemmen-Technik, die bereits für Bakteriorhodopsin verwendet worden war (Nagel et al. FEBS Letters, 377, 263-266 (1995); Nagel et al., Biophysical Journal 74, 403-412 (1998)) verschiedenen Experimenten unterworfen. Grünes Licht, nicht jedoch rotes Licht, induzierte einwärts gerichtete Ströme bei Oozyten, die eine der CHOP-1-RNAs exprimierten (Fig. 2). Das Auftreten von Photoströmen auch bei den kürzeren CHOP-1-RNAs demonstrierte, dass ein großer Teil des carboxyterminalen Bereichs von CHOP-1 für diese Funktion nicht erforderlich ist. Bei pH 6 und einer Transmembranspannung zwischen -100 und + 40 mV waren die Photoströme immer einwärts gerichtet (Fig. 3b). Der Ersatz von Chlorid durch Aspartat in der Lösung hatte keine nachweisbare Wirkung auf die Amplitude des Photostroms (Fig. 3a) oder dessen Strom-Spannungsverhältnis (Fig. 3b), ein Ergebnis, das Cl⁻ als transportiertes Ion ausschloss. Der Ersatz von Natrium durch N-Methyl-D-glucamin (oder von NaCl durch Sorbit, Daten nicht gezeigt) führte zu einem ähnlichen einwärts gerichteten Strom (Fig. 3a) ohne Veränderung des Strom-Spannungsverhältnisses (Fig. 3b), was anzeigt, dass Na⁺ von CHOP-1 nicht transportiert wird. Gleichermaßen ergab der Ersatz von Ca²⁺ durch Mg²⁺ keine Veränderung der Photoströme, ein Ergebnis, welches zeigte, dass Ca²⁺ ebenfalls nicht das transportierte Ion war (Fig. 3a,b).

Dagegen führte eine Erhöhung der Protonenkonzentration in der Badlösung, [H⁺]ₒ, auf pH-Werte von 5 bzw. 4 bei Potentialen zwischen -100 und +40 mV zu deutlichen Anstiegen der einwärts gerichteten Photoströme (Fig. 3a,b).

Die bisher erhaltenen Ergebnisse verweisen somit auf H⁺-lonen als Ladungsträger der lichtinduzierten Ströme. Aufgrund der eingangs erwähnten Sequenzhomologien zwischen dem CHOP-1-Protein und der Protonenpumpe Bakteriorhodopsin lag zunächst die Annahme nahe, dass auch das CHOP-1-Protein Bestandteil eines aktiven lonentransportsystems, nämlich einer Protonenpumpe wie Bakteriorhodopsin, wäre.

Während Bakteriorhodopsin jedoch bei allen getesteten Membranpotentialen von - 60 mV bis + 40 mV (siehe z.B. Nagel et al., Biophysical Journal 74, 403-412 (1998)) auch gegen einen bestehenden pH-Gradienten Protonen immer nach außen transportiert, war die Transportrichtung des CHOP-1-Systems abhängig von dem über die Membran vorliegenden pH-Gradienten und dem Membranpotential (Fig. 4a,b,c). Die gemessenen Umkehrpotentiale bei verschiedenen Ausgangs-pH-Gradienten (Fig. 4b,c) belegen eindeutig, dass die lichtinduzierten Ströme rein passiver Natur sind. Aus den beobachteten hohen Photoströmen ist zu schließen, dass der Protonentransport nicht nur durch eine erleichterte Diffusion der Protonen durch die Membran vonstatten geht, sondern dass das CHOP-Protein ein Protonenkanal ist.

Die Abhängigkeit des lichtinduzierten einwärts gerichteten Photostroms bei pH 5,5 und -40 mV von der Wellenlänge des Lichts ist in Fig. 4d dargestellt. Das Maximum in der Nähe von 500 nm entspricht den Aktionsspektren für Photorezeptorströme, für Phototaxie- und Photoschock-Reaktionen intakter *C. reinhardtii*-Zellen.

### BEISPIEL III

### Amplifizierung von CHOP-2-DNAs und Expression von funktionsfähigen CHOP-2-Photorezeptoren in Xenopus laevis

Eine CHOP-2-DNA vollständiger Länge, kodierend für die Aminosäuren 1-737, und eine C-terminal verkürzte CHOP-2-DNA, kodierend für die Aminosäuren 1-315 von CHOP-2, wurden von einer cDNA-Matrize vollständiger Länge mittels PCR unter Verwendung einer Polymerase mit Korrekturfunktion (pfu, Promega) und Primern, die BamHI- und HindIII-Restriktionsstellen enthielten, amplifiziert. Die Produkte wurden in den Vektor pGEMHE (Liman et al., Neuron 9, 861-71 (1992)) inseriert und in *E. coli* kloniert. Nach Überprüfung der Sequenzen wurden die CHOP-2-DNAs *in vitro* transkribiert (Ambion) und 20 bis 30 ng cRNA in Oozyten von *Xenopus laevis* injiziert, wie bereits für Bakteriorhodopsin beschrieben (Nagel et al., FEBS Letters, 377, 263-266 (1995). Die Oozyten, die das CHOP-2-Protein exprimierten, wurden mit einem *all-trans*-Retinal (1 µM) in Ringerscher Lösung zwei bis fünf Tage lang inkubiert.

### BEISPIEL IV

### Charakterisierung der CHOP-2-Photorezeptoren

Zur Untersuchung der lonentransportfunktion wurden die CHOP-2-exprimierenden Oozyten, die zur Bildung des ChR2 (Channelrhodopsin2)-lonenkanals in Ringer scher Lösung mit Retinal inkubiert worden waren, unter Anwendung ähnlicher Zweielektroden-Spannungsklemmen-Techniken, wie sie bereits für CHOP-1 bzw. ChR1 verwendet worden waren, verschiedenen Experimenten (Fig. 5-7) unterworfen.

Fig. 5 zeigt, dass der lichtinduzierte Photostrom bei Bestrahlung mit Dauerlicht (Wellenlänge 450 ± 25 nm; entspricht etwa dem Maximum des Aktionsspektrums) auf ein Gleichgewichtsniveau abfällt. Dies bedeutet, dass der ChR2-lonenkanal durch Dauerlicht dieser Wellenlänge desensibilisiert wird, möglicherweise durch eine Inaktivierung eines Teils der ChR2-Moleküle.

Fig. 6 demonstriert, dass der mit CHOP-2 gebildete lonenkanal im Gegensatz zu dem mit CHOP-1 gebildeten lonenkanal einen nicht-selektiven Kationenkanal darstellt, der neben Protonen auch verschiedene ein- und zweiwertige Kationen passieren lässt. Die Leitfähigkeit für die untersuchten einwertigen Kationen nimmt dabei in der Reihenfolge Li⁺ > Na⁺ > K⁺ > Cs⁺ >> TMA⁺ (Tetramethylammonium) = TEA⁺ (Tetraethylammonium), d.h., mit zunehmendem lonenradius, ab. Bei zweiwertigen Kationen nimmt die Leitfähigkeit ebenfalls mit zunehmenden lonenradius ab. Hier stellt jedoch Mg²⁺ die Ausnahme dar. ChR2 ist für Mg²⁺ nicht durchlässig, wahrscheinlich aufgrund der hohen Hydratationsenergie dieses Ions (Diebler et al., Pure Appl. Chem. 20, S. 93, 1969). Die jeweiligen Photoströme für den mit CHOP-2 voller Länge gebildeten lonenkanal unterschieden sich dabei nicht von den entsprechenden Photoströmen für den lonenkanal, der das CHOP-2-Fragment mit den aminoterminalen Aminosäuren 1-315 umfasst, was die Schlussfolgerung erlaubt, dass die Kationenleitfähigkeit durch den aminoterminalen Anteil des ChOP-2-Proteins, welcher die sieben mutmaßlichen Transmembrandomänen enthält, bestimmt wird.

SEQUENZPROTOKOLL

<110> Peter, Hegemann und Max-Planck-Gesellschaft zur Förderung der
Wissenschaften e. V.

<120> Verwendung von biologischen Photorezeptoren als direkt
lichtgesteuerte Ionenkanäle

<130> P33838-019

<160> 3

<170> PatentIn version 3.1

<210> 1

<211> 712

<212> PRT

<213> Chlamydomonas reinhardtii

<220>

<223> Aminosäuresequenz von CHOP-1 (AF385748) aus
Chlamydomonas reinhardtii

<400> 1

Met Ser Arg Arg Pro Trp Leu Leu Ala Leu Ala Leu Ala Val Ala Leu
1               5                   10                  15

Ala Ala Gly Ser Ala Gly Ala Ser Thr Gly Ser Asp Ala Thr Val Pro
            20                  25                  30

Val Ala Thr Gln Asp Gly Pro Asp Tyr Val Phe His Arg Ala His Glu
        35                  40                  45

Arg Met Leu Phe Gln Thr Ser Tyr Thr Leu Glu Asn Asn Gly Ser Val
    50                  55                  60

Ile Cys Ile Pro Asn Asn Gly Gln Cys Phe Cys Leu Ala Trp Leu Lys
65                  70                  75                  80

Ser Asn Gly Thr Asn Ala Glu Lys Leu Ala Ala Asn Ile Leu Gln Trp
                85                  90                  95

Ile Thr Phe Ala Leu Ser Ala Leu Cys Leu Met Phe Tyr Gly Tyr Gln
            100                 105                 110

Thr Trp Lys Ser Thr Cys Gly Trp Glu Glu Ile Tyr Val Ala Thr Ile
        115                 120                 125

Glu Met Ile Lys Phe Ile Ile Glu Tyr Phe His Glu Phe Asp Glu Pro
    130                 135                 140

Ala Val Ile Tyr Ser Ser Asn Gly Asn Lys Thr Val Trp Leu Arg Tyr
145                 150                 155                 160

Ala Glu Trp Leu Leu Thr Cys Pro Val Ile Leu Ile His Leu Ser Asn
                165                 170                 175

Leu Thr Gly Leu Ala Asn Asp Tyr Asn Lys Arg Thr Met Gly Leu Leu
            180                 185                 190

Val Ser Asp Ile Gly Thr Ile Val Trp Gly Thr Thr Ala Ala Leu Ser
        195                 200                 205

Lys Gly Tyr Val Arg Val Ile Phe Phe Leu Met Gly Leu Cys Tyr Gly
    210                 215                 220

Ile Tyr Thr Phe Phe Asn Ala Ala Lys Val Tyr Ile Glu Ala Tyr His
225                 230                 235                 240

Thr Val Pro Lys Gly Ile Cys Arg Asp Leu Val Arg Tyr Leu Ala Trp
                245                 250                 255

Leu Tyr Phe Cys Ser Trp Ala Met Phe Pro Val Leu Phe Leu Leu Gly
            260                 265                 270

Pro Glu Gly Phe Gly His Ile Asn Gln Phe Asn Ser Ala Ile Ala His
        275                 280                 285

Ala Ile Leu Asp Leu Ala Ser Lys Asn Ala Trp Ser Met Met Gly His
    290                 295                 300

Phe Leu Arg Val Lys Ile His Glu His Ile Leu Leu Tyr Gly Asp Ile
305                 310                 315                 320

Arg Lys Lys Gln Lys Val Asn Val Ala Gly Gln Glu Met Glu Val Glu
                325                 330                 335

Thr Met Val His Glu Glu Asp Asp Glu Thr Gln Lys Val Pro Thr Ala
            340                 345                 350

Lys Tyr Ala Asn Arg Asp Ser Phe Ile Ile Met Arg Asp Arg Leu Lys
        355                 360                 365

Glu Lys Gly Phe Glu Thr Arg Ala Ser Leu Asp Gly Asp Pro Asn Gly
    370                 375                 380

Asp Ala Glu Ala Asn Ala Ala Ala Gly Gly Lys Pro Gly Met Glu Met
385                 390                 395                 400

Gly Lys Met Thr Gly Met Gly Met Gly Met Gly Ala Gly Met Gly Met
                405                 410                 415

Ala Thr Ile Asp Ser Gly Arg Val Ile Leu Ala Val Pro Asp Ile Ser
            420                 425                 430

Met Val Asp Phe Phe Arg Glu Gln Phe Ala Arg Leu Pro Val Pro Tyr
        435                 440                 445

Glu Leu Val Pro Ala Leu Gly Ala Glu Asn Thr Leu Gln Leu Val Gln
    450                 455                 460

Gln Ala Gln Ser Leu Gly Gly Cys Asp Phe Val Leu Met His Pro Glu
465                 470                 475                 480

Phe Leu Arg Asp Arg Ser Pro Thr Gly Leu Leu Pro Arg Leu Lys Met
                485                 490                 495

Gly Gly Gln Arg Ala Ala Ala Phe Gly Trp Ala Ala Ile Gly Pro Met
            500                 505                 510

Arg Asp Leu Ile Glu Gly Ser Gly Val Asp Gly Trp Leu Glu Gly Pro
        515                 520                 525

Ser Phe Gly Ala Gly Ile Asn Gln Gln Ala Leu Val Ala Leu Ile Asn
    530                 535                 540

Arg Met Gln Gln Ala Lys Lys Met Gly Met Met Gly Gly Met Gly Met
545                 550                 555                 560

Gly Met Gly Gly Gly Met Gly Met Gly Met Gly Met Gly Met Gly Met
                565                 570                 575

Ala Pro Ser Met Asn Ala Gly Met Thr Gly Gly Met Gly Gly Ala Ser
            580                 585                 590

Met Gly Gly Ala Val Met Gly Met Gly Met Gly Met Gln Pro Met Gln
        595                 600                 605

Gln Ala Met Pro Ala Met Ser Pro Met Met Thr Gln Gln Pro Ser Met
    610                 615                 620

Met Ser Gln Pro Ser Ala Met Ser Ala Gly Gly Ala Met Gln Ala Met
625                 630                 635                 640

Gly Gly Val Met Pro Ser Pro Ala Pro Gly Gly Arg Val Gly Thr Asn
                645                 650                 655

Pro Leu Phe Gly Ser Ala Pro Ser Pro Leu Ser Ser Gln Pro Gly Ile
            660                 665                 670

Ser Pro Gly Met Ala Thr Pro Pro Ala Ala Thr Ala Ala Pro Ala Ala
        675                 680                 685

Gly Gly Ser Glu Ala Glu Met Leu Gln Gln Leu Met Ser Glu Ile Asn
    690                 695                 700

Arg Leu Lys Asn Glu Leu Gly Glu
705                 710

<210>  2

<211>  737

<212>  PRT

<213>  Chlamydomonas reinhardtii

<220>

<223>  Aminosäuresequenz von CHOP-2 (AF461397) aus
       Chlamydomonas reinhardtii

<400> 2

Met Asp Tyr Gly Gly Ala Leu Ser Ala Val Gly Arg Glu Leu Leu Phe
1               5                   10                  15

Val Thr Asn Pro Val Val Val Asn Gly Ser Val Leu Val Pro Glu Asp
            20                  25                  30

Gln Cys Tyr Cys Ala Gly Trp Ile Glu Ser Arg Gly Thr Asn Gly Ala
        35                  40                  45

Gln Thr Ala Ser Asn Val Leu Gln Trp Leu Ala Ala Gly Phe Ser Ile
    50                  55                  60

Leu Leu Leu Met Phe Tyr Ala Tyr Gln Thr Trp Lys Ser Thr Cys Gly
65                  70                  75                  80

Trp Glu Glu Ile Tyr Val Cys Ala Ile Glu Met Val Lys Val Ile Leu
                85                  90                  95

Glu Phe Phe Phe Glu Phe Lys Asn Pro Ser Met Leu Tyr Leu Ala Thr
            100                 105                 110

Gly His Arg Val Gln Trp Leu Arg Tyr Ala Glu Trp Leu Leu Thr Cys
        115                 120                 125

Pro Val Ile Leu Ile His Leu Ser Asn Leu Thr Gly Leu Ser Asn Asp
    130                 135                 140

Tyr Ser Arg Arg Thr Met Gly Leu Leu Val Ser Asp Ile Gly Thr Ile
145                 150                 155                 160

Val Trp Gly Ala Thr Ser Ala Met Ala Thr Gly Tyr Val Lys Val Ile
                165                 170                 175

Phe Phe Cys Leu Gly Leu Cys Tyr Gly Ala Asn Thr Phe Phe His Ala
            180                 185                 190

Ala Lys Ala Tyr Ile Glu Gly Tyr His Thr Val Pro Lys Gly Arg Cys
        195                 200                 205

Arg Gln Val Val Thr Gly Met Ala Trp Leu Phe Phe Val Ser Trp Gly
    210                 215                 220

Met Phe Pro Ile Leu Phe Ile Leu Gly Pro Glu Gly Phe Gly Val Leu
225                 230                 235                 240

Ser Val Tyr Gly Ser Thr Val Gly His Thr Ile Ile Asp Leu Met Ser
                245                 250                 255

Lys Asn Cys Trp Gly Leu Leu Gly His Tyr Leu Arg Val Leu Ile His
            260                 265                 270

Glu His Ile Leu Ile His Gly Asp Ile Arg Lys Thr Thr Lys Leu Asn
        275                 280                 285

Ile Gly Gly Thr Glu Ile Glu Val Glu Thr Leu Val Glu Asp Glu Ala
    290                 295                 300

Glu Ala Gly Ala Val Asn Lys Gly Thr Gly Lys Tyr Ala Ser Arg Glu
305                 310                 315                 320

Ser Phe Leu Val Met Arg Asp Lys Met Lys Glu Lys Gly Ile Asp Val
                325                 330                 335

Arg Ala Ser Leu Asp Asn Ser Lys Glu Val Glu Gln Glu Gln Ala Ala
            340                 345                 350

Arg Ala Ala Met Met Met Met Asn Gly Asn Gly Met Gly Met Gly Met
        355                 360                 365

Gly Met Asn Gly Met Asn Gly Met Gly Gly Met Asn Gly Met Ala Gly
    370                 375                 380

Gly Ala Lys Pro Gly Leu Glu Leu Thr Pro Gln Leu Gln Pro Gly Arg
385                 390                 395                 400

Val Ile Leu Ala Val Pro Asp Ile Ser Met Val Asp Phe Phe Arg Glu
                405                 410                 415

Gln Phe Ala Gln Leu Ser Val Thr Tyr Glu Leu Val Pro Ala Leu Gly
            420                 425                 430

Ala Asp Asn Thr Leu Ala Leu Val Thr Gln Ala Gln Asn Leu Gly Gly
        435                 440                 445

Val Asp Phe Val Leu Ile His Pro Glu Phe Leu Arg Asp Arg Ser Ser
    450                 455                 460

Thr Ser Ile Leu Ser Arg Leu Arg Gly Ala Gly Gln Arg Val Ala Ala
465                 470                 475                 480

Phe Gly Trp Ala Gln Leu Gly Pro Met Arg Asp Leu Ile Glu Ser Ala
                485                 490                 495

Asn Leu Asp Gly Trp Leu Glu Gly Pro Ser Phe Gly Gln Gly Ile Leu
            500                 505                 510

Pro Ala His Ile Val Ala Leu Val Ala Lys Met Gln Gln Met Arg Lys
        515                 520                 525

Met Gln Gln Met Gln Gln Ile Gly Met Met Thr Gly Gly Met Asn Gly
    530                 535                 540

Met Gly Gly Gly Met Gly Gly Gly Met Asn Gly Met Gly Gly Gly Asn
545                 550                 555                 560

Gly Met Asn Asn Met Gly Asn Gly Met Gly Gly Gly Met Gly Asn Gly
                565                 570                 575

Met Gly Gly Asn Gly Met Asn Gly Met Gly Gly Gly Asn Gly Met Asn
            580                 585                 590

Asn Met Gly Gly Asn Gly Met Ala Gly Asn Gly Met Gly Gly Gly Met
        595                 600                 605

Gly Gly Asn Gly Met Gly Gly Ser Met Asn Gly Met Ser Ser Gly Val
    610                 615                 620

Val Ala Asn Val Thr Pro Ser Ala Ala Gly Gly Met Gly Gly Met Met
625                 630                 635                 640

Asn Gly Gly Met Ala Ala Pro Gln Ser Pro Gly Met Asn Gly Gly Arg
                645                 650                 655

Leu Gly Thr Asn Pro Leu Phe Asn Ala Ala Pro Ser Pro Leu Ser Ser
            660                 665                 670

Gln Leu Gly Ala Glu Ala Gly Met Gly Ser Met Gly Gly Met Gly Gly
        675                 680                 685

Met Ser Gly Met Gly Gly Met Gly Gly Met Gly Gly Met Gly Gly Ala
    690                 695                 700

Gly Ala Ala Thr Thr Gln Ala Ala Gly Gly Asn Ala Glu Ala Glu Met
705                 710                 715                 720

Leu Gln Asn Leu Met Asn Glu Ile Asn Arg Leu Lys Arg Glu Leu Gly
                725                 730                 735

Glu

<210> 3

<211> 259

<212> PRT

<213> Halobacterium salinarum

<220>

<223> Aminosäuresequenz von Bakteriorhodopsin aus
      Halobakterium salinarum

<400> 3

Met Leu Pro Thr Ala Val Glu Gly Val Ser Gln Ala Gln Ile Thr Gly
1               5                   10                  15

Arg Pro Glu Trp Ile Trp Leu Ala Leu Gly Thr Ala Leu Met Gly Leu
            20                  25                  30

Gly Thr Leu Tyr Phe Leu Val Lys Gly Met Gly Val Ser Asp Pro Asp
        35                  40                  45

Ala Lys Lys Phe Tyr Ala Ile Thr Thr Leu Val Pro Ala Ile Ala Phe
    50                  55                  60

Thr Met Tyr Leu Ser Met Leu Leu Gly Tyr Gly Leu Thr Met Val Pro
65                  70                  75                  80

Phe Gly Gly Glu Gln Asn Pro Ile Tyr Trp Ala Arg Tyr Ala Asp Trp
                85                  90                  95

Leu Phe Thr Thr Pro Leu Leu Leu Leu Asp Leu Ala Leu Leu Val Asp
            100                 105                 110

Ala Asp Gln Gly Thr Ile Leu Ala Leu Val Gly Ala Asp Gly Ile Met

115                 120                 125

Ile Gly Thr Gly Leu Val Gly Ala Leu Thr Lys Val Tyr Ser Tyr Arg
    130                 135                 140

Phe Val Trp Trp Ala Ile Ser Thr Ala Ala Met Leu Tyr Ile Leu Tyr
145                 150                 155                 160

Val Leu Phe Phe Gly Phe Thr Ser Lys Ala Glu Ser Met Arg Pro Glu
                165                 170                 175

Val Ala Ser Thr Phe Lys Val Leu Arg Asn Val Thr Val Val Leu Trp
            180                 185                 190

Ser Ala Tyr Pro Val Val Trp Leu Ile Gly Ser Glu Gly Ala Gly Ile
        195                 200                 205

Val Pro Leu Asn Ile Glu Thr Leu Leu Phe Met Val Leu Asp Val Ser
    210                 215                 220

Ala Lys Val Gly Phe Gly Leu Ile Leu Leu Arg Ser Arg Ala Ile Phe
225                 230                 235                 240

Gly Glu Ala Glu Ala Pro Glu Pro Ser Ala Gly Asp Gly Ala Ala Ala
                245                 250                 255

Thr Ser Asp

Aminosäuresequenz der CHOP2-315/H134R-Mutante:

## Patentansprüche

1. Verwendung eines biologischen Photorezeptors als direkt lichtgesteuerter lonenkanal zur Veränderung der lonenleitfähigkeit einer Membran mit Hilfe von Licht,
- bei welcher der Photorezeptor ein Apoprotein und ein lichtsensitives Polyen aufweist, wobei das lichtsensitive Polyen kovalent an das Apoprotein gebunden ist, mit dem Apoprotein wechselwirkt und als lichtempfindliches Tor fungiert,
- bei welcher das Apoprotein ein lonenkanal bildendes Opsin oder ein Derivat oder Fragment eines lonenkanal bildenden Opsins ist und
- bei welcher durch Belichtung mittels Veränderung der lonenleitfähigkeit der Membran der Tod einer Zelle hervorgerufen werden kann oder hervorgerufen wird.

2. Verwendung nach Anspruch 1,
bei welcher der biologische Fotorezeptor in der Membran einer Organelle einer Zelle exprimiert wird.

3. Verwendung nach einem der vorangehenden Ansprüche,
bei welcher der biologische Fotorezeptor in der Membran von Mitochondrien einer Zelle und/oder in der Membran des endoplasmatischen Retikulums einer Zelle exprimiert wird.

4. Verwendung nach einem der vorangehenden Ansprüche,
bei welcher der biologische Fotorezeptor selektiv exprimiert wird, insbesondere in Bezug auf ein oder mehrere Gewebe, Zelltypen, insbesondere selektiv in Tumorzellen, und/oder Organe.

5. Verwendung nach einem der vorangehenden Ansprüche,
wobei die Expression durch virale Transfektion erfolgt.

6. Verwendung nach einem der vorangehenden Ansprüche,
wobei das Zuführen von Licht selektiv erfolgt.

7. Verwendung nach einem der vorangehenden Ansprüche,
in einem Verfahren, Kit und/oder System zur Behandlung eines oder mehrerer Zelltypen, eines oder mehrerer Gewebe, eines oder mehrerer Organe, eines oder mehrerer Organismen und/oder des menschlichen Körpers.

8. Verwendung nach einem der vorangehenden Ansprüche,
wobei das Apoprotein ein Transmembranprotein mit 5 oder mehr Transmembranhelices ist.

9. Verwendung nach einem der vorangehenden Ansprüche,
wobei das Apoprotein ein Opsin-Protein oder ein Derivat oder Fragment eines natürlich vorkommenden Opsin-Proteins ist, insbesondere als Ergebnis eines Austausches und/oder einer Insertion und/oder Deletion von einer oder mehreren Aminosäure(n) in der natürlichen Aminosäuresequenz des Opsin-Proteins ist.

10. Verwendung nach einem der vorangehenden Ansprüche,
wobei das Apoprotein aus niederen Pflanzen stammt, insbesodnere aus Algen.

11. Verwendung nach Anspruch 10,
wobei das Apoprotein ein Opsin-Protein aus Chlamydomonas reinhardtii ist.

12. Verwendung nach einem der vorangehenden Ansprüche,
wobei das Apoprotein mindestens die Aminosäuren 61 bis 310 des Channelopsin1 (CHOP-1) gemäß SEQ ID NO:AF385748 (National Center for Biotechnology Information, NCBI) umfasst.

13. Verwendung nach einem der vorangehenden Ansprüche 1 bis 24,
wobei das Apoprotein mindestens die Aminosäuren 24 bis 268 des Channelopsin2 (CHOP-2) gemäß SEQ ID NO:AF461397 umfasst,
wobei insbesondere die Aminosäure Histidin an Position 134 des Channelopsin2 gemäß SEQ ID NO:AF461397 durch eine andere Aminosäure ersetzt ist oder
wobei die Aminosäure Histidin an Position 134 durch Arginin ersetzt ist.

14. Verwendung nach einem der Ansprüche 1 bis 9,
wobei das Opsin-Protein von Protozoen, Bakterien, Archaeen oder Pilzen stammt.

15. Verwendung nach einem der vorangehenden Ansprüche,
wobei das lichtsensitive Polyen ein Retinal oder Retinalderivat ist,
wobei das Retinalderivat insbesondere aus der folgenden Gruppe ausgewählt ist: 3,4-Dehydroretinal, 13-Ethylretinal, 9-dm-Retinal, 3-Hydroxyretinal, 4-Hydroxyretinal, Naphthylretinal; 3,7,11-Trimethyl-dodeca-2,4,6,8,10-pentaenal; 3,7-Dimethyl-deca-2,4,6,8-tetraenal; 3,7-Dimethyl-octa-2,4,6-trienal; sowie 6-7- oder 8-9- oder 10-11-Rotations-blockierte Retinale.
